# EUROPEAN PATENT SPECIFICATION

(11) **EP 3 099 262 B1**
(45) Date of publication and mention of the grant of the patent: **23.02.2022**
(21) Application number: 15742899.6
(22) Date of filing: 30.01.2015
(51) Int. Cl.: A61B 18/02, A61B 18/04, A61B 18/18, A61F 7/00, A61B 18/00, A61F 7/02, A61N 5/06, A61N 7/00, A61B 18/12, A61B 18/20, A61N 5/067

(54) **COOLING DEVICE TO DISRUPT FUNCTION SEBACEOUS GLANDS**
KÜHLVORRICHTUNG ZUR UNTERBRECHUNG DER FUNKTION DER TALGDRÜSEN
DISPOSITIF DE REFROIDISSEMENT POUR PERTURBER LE FONCTIONNEMENT DES GLANDES SÉBACÉES

(30) Priority: 31.01.2014 US 201461934654 P
(43) Date of publication of application: 07.12.2016
(73) Proprietor: The General Hospital Corporation, Boston, MA 02114 (US)
(72) Inventor: ANDERSON, Richard Rox, Boston, Massachusetts 02114 (US); TAM, Joshua, Boston, Massachusetts 02114 (US); JALIAN, Hrak Ray, Glendale, California 91202 (US)
(74) Representative: Forresters IP LLP
(86) International application number: PCT/US2015/013918
(87) International publication number: WO 2015/117005

(56) References cited:
- US-A1- 2007 010 861
- US-A1- 2007 010 861
- US-A1- 2007 255 362
- US-A1- 2010 049 178
- US-B2- 6 743 222
- US-B2- 7 276 058
- US-B2- 7 276 058
- US-B2- 8 372 130
- US-B2- 8 372 130
- PAITHANKAR, DY ET AL.: 'Acne Treatment With a 1,450 nm Wavelength Laser and Cryogen Spray . Cooling.' LASERS IN SURGERY AND MEDICINE vol. 31, no. 2, 2002, pages 106 - 114, XP055294809
- WILLIAMS, M ET AL.: 'The effect of local temperature changes on sebum excretion rate and forehead surface lipid composition.' BRITISH JOURNAL OF DERMATOLOGY. vol. 88, no. 3, March 1973, pages 257 - 262, XP055357414

## Description

### BACKGROUND

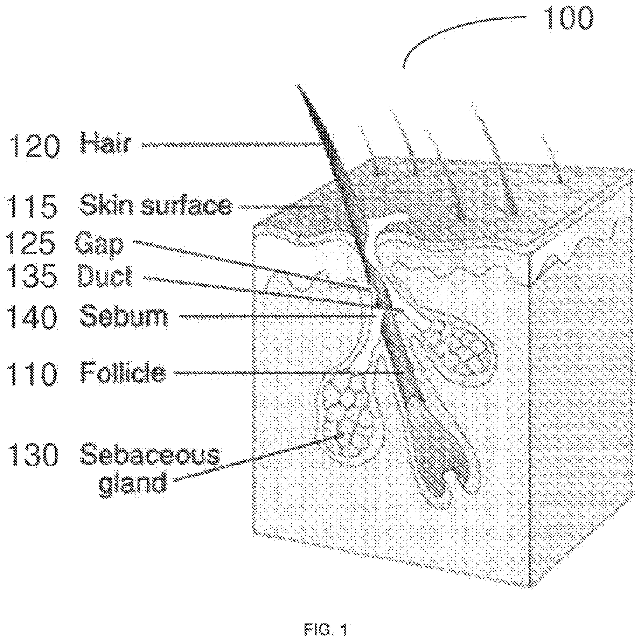

FIG. 1 shows a hair follicle structure 100, which includes a hair follicle 110, hair shaft 120 and one or more associated sebaceous glands 130. A small gap 125 is generally present between the hair shaft 120 and the sides of the follicle 110 in the upper portion of the follicle structure 100 leading to the skin surface 115. One or more sebaceous glands 130 are often connected to this gap via a duct 135, as shown in FIG. 1.

Sebaceous glands 130 secrete an oily substance called sebum 140, which is a mixture of lipids and debris from dead cells shed by the gland 130. The sebum 140 is deposited into the upper portion (infundibulum) of the hair follicle 110, and eventually may rise up to the skin surface 115. Sebum 140 can lubricate and protect hair shafts 120, act as a moisturizer, and can also help to seal the follicle opening 125 from external substances.

Sebum accumulation and/or blockage of the duct 135 between the sebaceous gland 130 and the hair follicle 110 by sebum 140 can be a major cause of the common skin condition *acne vulgaris* (generally referred to as "acne"). Sebum blockages can enlarge to form blackheads or whiteheads. Redness, swelling, and infection may also occur. These acne symptoms can be aesthetically undesirable and socially stigmatizing, and they can also lead to the formation of disfiguring scars.

Present approaches for alleviating acne symptoms and avoiding more serious complications include application of topical solutions of substances such as benzoyl peroxide (available over-the-counter or by prescription) or salicylic acid, or prescription antibiotics such as erythromycin, clindamycin, or tetracycline derivatives. Topical retinoids can also be applied to alleviate acne symptoms. Retinoids include tretinoin (brand name RETIN-A^{®}), adapalene, and tazarotene and the non-prescription retinol or derivatives. Retinoids appear to influence cells in the follicle lining, which can help to prevent hyperkeratinization of these cells that can lead to blockages.

The effectiveness of such topical treatments, particularly solutions containing retinoids, may generally be limited by the ability of the topical solutions to penetrate deeply into the follicle 110 and into the sebaceous gland 130 itself. Accumulation of sebum 140 and debris in the hair follicle 110 and/or duct 135 leading into the sebaceous gland 130 can inhibit penetration of topical solutions into the follicle region where they may be more efficacious.

Oral intake of isotretinoin, a retinoid (available under the brand names ACCUTANE^{®}, SOTRET^{®}, CLARUS^{™}, etc.) has been shown to provide long-term reduction of acne symptoms or severity thereof. Isotretinoin can be very effective for treating severe cases of acne. However, oral administration of isotretinoin is believed to cause significant side effects, including liver damage and birth defects when used by pregnant women. For these reasons, treatment of acne using oral administration of isotretinoin generally requires close monitoring and a specified timetable for treatment cycles.

Other techniques that can be used to treat acne with varying degrees of effectiveness include phototherapy (e.g., photodynamic therapy ("PDT") or treatment with various lasers or intense pulsed light sources). PDT generally involves topical application of a solution containing 5-aminolevulinic acid (ALA) or other photosensitizer precursors or photosensitizers, followed by irradiation with optical energy to activate the photosensitizer and selectively affect certain tissues where the photosensitizer is present.

Each of these treatments for acne poses various costs and side effects. Accordingly, there remains a need for new and improved approaches for treating acne and other sebaceous gland disorders.

### SUMMARY OF THE INVENTION

The disclosure provides a cooling device including: a cooling unit; and a control unit programmed to control operation of the cooling unit in order to cool one or more sebaceous glands within a local region for a period of time and to a temperature not higher than 15° C, sufficient to disrupt function of the one or more sebaceous glands without permanently injuring epidermal tissue or cooling subcutaneous adipose tissue to 25° C or below 25° C. The control unit can be programmed to control operation of the cooling unit to cool the one or more sebaceous glands below a temperature selected from the group consisting of: about 15° C, about 14° C, about 13° C, about 12° C, about 11° C, about 10° C, about 9° C, about 8° C, about 7° C, about 6° C, about 5° C, about 4° C, about 3° C, about 2° C, about 1° C, about 0° C, about -1° C, about -2° C, about -3° C, about -4° C, about -5° C, about -6° C, about -7° C, about -8° C, about -9° C, and about -10° C.

The control unit can be programmed to control operation of the cooling unit to cool the sebaceous glands at a rate greater than 20° C per minute. The control unit can be programmed to control operation of the cooling unit to cool the sebaceous glands at a rate selected from the group consisting of: between about 20° C per minute and about 30° C per minute, between about 30° C per minute and about 40° C per minute, between about 40° C per minute and about 50° C per minute, and between about 50° C per minute and about 60° C per minute. The control unit can be programmed to control operation of the cooling unit to cool the sebaceous glands at a rate selected from the group consisting of: between about 1° C per second and about 2° C per second, between about 2° C per second and about 3° C per second, between about 3° C per second and about 4° C per second, between about 4° C per second and about 5° C per second, between about 5° C per second and about 6° C per second, between about 6° C per second and about 7° C per second, between about 7° C per second and about 8° C per second, between about 8° C per second and about 9° C per second, and between about 9° C per second and about 10° C per second.

The period of time can be selected from the group consisting of: less than about 10 seconds, between about 10 seconds and about 20 seconds, between about 20 seconds and about 30 seconds, and between about 30 seconds and about 40 seconds. The period of time can be selected from the group consisting of: between about 40 seconds and about 1 minute, between about 1 minute and about 2 minutes, and between about 2 minutes and about 5 minutes.

The control unit can be programmed to control operation of the cooling unit to: cool the one or more sebaceous glands for a first period of time; actively or passively warm the one or more sebaceous glands; and cool the one or more sebaceous glands for a second period of time. The one or more sebaceous glands can be warmed by suspending or reducing operation of the cooling unit. The one or more sebaceous glands can be warmed by controlling the cooling unit to actively raise the temperature of the cooling unit.

The cooling unit can further include an energy source. The one or more sebaceous glands can be warmed by actuation of the energy source. The control unit can be programmed to control operation of the energy source to warm the sebaceous glands at a rate greater than 20° C per minute. The control unit can be programmed to control operation of the energy source to warm the sebaceous glands at a rate selected from the group consisting of: between about 20° C per minute and about 30° C per minute, between about 30° C per minute and about 40° C per minute, between about 40° C per minute and about 50° C per minute, and between about 50° C per minute and about 60° C per minute. The control unit can be programmed to control operation of the energy source to warm the sebaceous glands at a rate selected from the group consisting of: between about 1° C per second and about 2° C per second, between about 2° C per second and about 3° C per second, between about 3° C per second and about 4° C per second, between about 4° C per second and about 5° C per second, between about 5° C per second and about 6° C per second, between about 6° C per second and about 7° C per second, between about 7° C per second and about 8° C per second, between about 8° C per second and about 9° C per second, and between about 9° C per second and about 10° C per second.

The energy source can be adapted and configured to apply energy to the subcutaneous adipose tissue. The period of time can be selected from the group consisting of: less than about 5 minutes, between about 5 minutes and about 10 minutes, between about 10 minutes and about 15 minutes, between about 15 minutes and about 20 minutes, between about 20 minutes and about 25 minutes, between about 25 minutes and about 30 minutes, between about 30 minutes and about 35 minutes, between about 35 minutes and about 40 minutes, between about 40 minutes and about 45 minutes, between about 45 minutes and about 50 minutes, between about 50 minutes and about 55 minutes, and between about 55 minutes and about 60 minutes.

The control unit can be programmed to control operation of the cooling unit to cool the one or more sebaceous glands below a temperature selected from the group consisting of: about -11° C, about -12° C, about -13° C, about -14° C, about -15° C, about -16° C, about -17° C, about -18° C, about -19° C, about -20° C, about -21° C, about -22° C, about -23° C, about -24° C, about -25° C, about -26° C, about -27° C, about -28° C, about -29° C, and about -30° C.

The energy source can be selected from the group consisting of: a light source, a resistive heater, a Peltier element, an RF source, a microwave source, an ultrasound source, and a heated liquid.

The control unit can be further programmed to: tighten one or more constriction elements in order to reduce blood flow to the local region during the cooling steps; and loosen the one or more constriction elements in order to restore normal blood flow to the local region during the warming step.

The control unit can be programmed to control operation of the cooling unit to subject the one or more sebaceous glands to a plurality of cooling-warming cycles. The plurality of cooling-warming cycles can be selected from the group consisting of: 2, 3, 4, 5, 6, 7, 8, 9, and 10.

The cooling device can further include a treatment interface in thermal communication with the cooling unit. The treatment interface can be adapted and configured for application to the local region. The treatment interface can be a mask having a complementary surface that substantially approximates a human face. The treatment interface can have a convex surface or a concave surface. The cooling device can further include a vacuum in communication with the treatment interface. The vacuum can be adapted and configured to generate reduced pressure in order to hold the treatment interface against the local region.

The cooling device includes one or more feedback devices in communication with the control unit. The control unit contains computer program code including instructions for controlling operation of the cooling unit in order to cool one or more sebaceous glands within a local region for a period of time and to a temperature sufficient to at least temporarily disrupt function of the one or more sebaceous glands without permanently injuring epidermal tissue or cooling subcutaneous adipose tissue to 25° C or below 25° C.

Another aspect of the invention provides a kit including: a cooling device as described herein; an intermediary material; and instructions to apply the intermediary material to the local region prior to application of the cooling device.

This aspect of the invention can have a variety of embodiments. The intermediary material can enhance thermal conductivity between the cooling device. The intermediary material can be a cryoprotectant. The intermediary material can include one or more selected from the group consisting: water, heavy water, oil, peanut oil, glycerol, glycol, polypropylene glycol (PPG), polyethylene glycol (PEG), propylene glycol, ethylene glycol, dimethyl sulfoxide (DMSO), alcohol, ethanol, propanol, iso-propanol, carboxyl polyethylene polymer, hydroxyethyl xylose polymer, carboxyl methylcellulose, and hydroxyethyl cellulose (HEC).

Another aspect of the disclosure provides a method, for disrupting function of one or more sebaceous glands within a local region of a subject. The method includes: applying a cooling device to the local region of the subject; and modulating operation of the cooling device or periodically removing the cooling device in order to cool one or more sebaceous glands within the local region for a period of time and to a temperature sufficient to disrupt function of the one or more sebaceous glands without permanently injuring epidermal tissue or cooling subcutaneous adipose tissue to 25° C or below 25° C.

This aspect of the disclosure can have a variety of embodiments. The subject can be a human. The local region can be selected from the group consisting of: a face, a chest and a back. The local region of the subject can be diagnosed with a sebaceous gland disorder.

A rate of sebum secretion can be temporarily reduced after performance of the method relative to a rate of sebum secretion prior to performance of the method. The rate of sebum secretion can be reduced by a percentage selected from the group consisting of: less than about 10%, between about 10% and about 20%, between about 20% and about 30%, between about 30% and about 40%, between about 40% and about 50%, between about 50% and about 60%, between about 60% and about 70%, between about 70% and about 80%, between about 80% and about 90%, and between about 90% and about 100%.

A quantity of sebum secretion can be temporarily reduced after performance of the method relative to a quantity of sebum secretion prior to performance of the method. The rate of sebum secretion can be reduced by a percentage selected from the group consisting of: less than about 10%, between about 10% and about 20%, between about 20% and about 30%, between about 30% and about 40%, between about 40% and about 50%, between about 50% and about 60%, between about 60% and about 70%, between about 70% and about 80%, between about 80% and about 90%, and between about 90% and about 100%.

Function of the one or more sebaceous glands can be disrupted for a period selected from the group consisting of: at least about 1 hour, at least about 2 hours, at least about 3 hours, at least about 4 hours, at least about 6 hours, at least about 8 hours, at least about 12 hours, at least about 18 hours, at least about 1 day, at least about 2 days, at least about 3 days, at least about 4 days, at least about 5 days, a least about 6 days, at least about 1 week, at least about 2 weeks, at least about 3 weeks, and at least about 4 weeks. Function of the one or more sebaceous glands can be permanently disrupted after performance of the method.

The one or more sebaceous glands can be cooled below a temperature selected from the group consisting of: about 15° C, about 14° C, about 13° C, about 12° C, about 11° C, about 10° C, about 9° C, about 8° C, about 7° C, about 6° C, about 5° C, about 4° C, about 3° C, about 2° C, about 1° C, about 0° C, about -1° C, about -2° C, about -3° C, about -4° C, about -5° C, about -6° C, about -7° C, about -8° C about -9° C and about -10° C.

The one or more sebaceous glands can be cooled at a rate greater than 20° C. The one or more sebaceous glands can be cooled at a rate selected from the group consisting of: between 20° C per minute and about 30° C per minute, between 30° C per minute and about 40° C per minute, between 40° C per minute and about 50° C per minute, and between 50° C per minute and about 60° C per minute. The one or more sebaceous glands can be cooled at a rate selected from the group consisting of: between about 1° C per second and about 2° C per second, between about 2° C per second and about 3° C per second, between about 4° C per second and about 5° C per second, between about 5° C per second and about 6° C per second, between about 6° C per second and about 7° C per second, between about 7° C per second and about 8° C per second, between about 8° C per second and about 9° C per second, between about 9° C per second and about 10° C per second.

The one or more sebaceous glands can be cooled for a period selected from the group consisting of: less than about 10 seconds, between about 10 seconds and about 20 seconds, between about 20 seconds and about 30 seconds, and between about 30 seconds and about 40 seconds. The one or more sebaceous glands can be cooled for a period selected from the group consisting of: between 4-seconds and about 1 minute and between about 1 minute and about 2 minutes, and between about 2 minutes and about 5 minutes.

The step of modulating operation of the cooling device or periodically removing the cooling device can further include: cooling the one or more sebaceous glands for a first period of time; actively or passively warming the one or more sebaceous glands; and cooling the one or more sebaceous glands for a second period of time. The actively or passively warming step can include one or more selected from the group consisting of: suspending or reducing operation of the cooling device, controlling the cooling device to actively raise the temperature of the cooling device, and actuating an energy source to introduce energy into the local region.

The sebaceous glands can be warmed at a rate greater than 20° C. The one or more sebaceous glands can be warmed at a rate selected from the group consisting of: between 20° C per minute and about 30° C per minute, between 30° C per minute and about 40° C per minute, between 40° C per minute and about 50° C per minute, and between 50° C per minute and about 60° C per minute. The one or more sebaceous glands can be warmed at a rate selected from the group consisting of: between about 1° C per second and about 2° C per second, between about 2° C per second and about 3° C per second, between about 4° C per second and about 5° C per second, between about 5° C per second and about 6° C per second, between about 6° C per second and about 7° C per second, between about 7° C per second and about 8° C per second, between about 8° C per second and about 9° C per second, between about 9° C per second and about 10° C per second.

The method can further include applying energy to the subcutaneous adipose tissue. The one or more sebaceous glands can be cooled for a period selected from the group consisting of: less than about 5 minutes, between 5 minutes and about 10 minutes, between 10 minutes and about 15 minutes, between 15 minutes and about 20 minutes, between 20 minutes and about 25 minutes, between 25 minutes and about 30 minutes, between 30 minutes and about 35 minutes, between 35 minutes and about 40 minutes, between 40 minutes and about 45 minutes, between 45 minutes and about 50 minutes, between 50 minutes and about 55 minutes, and between 55 minutes and about 60 minutes.

The one or more sebaceous glands can be cooled below a temperature selected from the group consisting of: about -11, about -12° C, about -13° C, about - 14° C, about -15° C, about -16° C, about -17° C, about -18° C, about -19° C, about - 20° C, about -21° C, about -22° C, about -23° C, about -24° C, about -25° C, about - 26° C, about -27° C, about -28° C, about -29° C and about -30° C.

The method can further include applying an intermediary material to the local region prior to application of the cooling device.

Another aspect of the disclosure provides a method, for reducing an abnormally elevated rate of sebum secretion from one or more sebaceous glands within a local region of a subject. The method includes: applying a cooling device to the local region of the subject; and modulating operation of the cooling device or periodically removing the cooling device in order to cool one or more sebaceous glands within the local region for a period of time and to a temperature sufficient to disrupt function of the one or more sebaceous glands without permanently injuring epidermal tissue, thereby reducing the abnormally elevated rate of sebum secretion from the one or more sebaceous glands within the local region.

This aspect of the disclosure can have a variety of embodiments. The local region of the subject can be diagnosed with a sebaceous gland disorder. The local region can be selected from the group consisting of: a back and a chest. The local region can be a face.

### DESCRIPTION OF THE FIGURES

For a fuller understanding of the nature and desired objects of the present invention, reference is made to the following detailed description taken in conjunction with the following figures.
FIG. 1 depicts an exemplary hair follicle structure 100.
FIG. 2 depicts a method 200 of disrupting sebaceous glands according to the disclosure.
FIG. 3 depicts a cooling device 300 according to the disclosure.
FIG. 4 depicts a kit 400 according to the disclosure.
FIG. 5 depicts a cooling device 500 according to the disclosure.
FIGS. 6A-6D depict various cooling profiles according to the disclosure.
FIG. 7 provides a schematic of a cooling apparatus utilized in murine experiments described the Working Examples section.
FIG. 8 provides gross and histologic views of murine ears at various time points following cooling at -7° C for 10 minutes. Retraction of lipid-laden sebocytes from peripheral cells is visible at day two. By day three, there is an eosinophilic collection of material within the gland lumen, which is a remnant of the dead and dying sebocytes induced by the treatment. By day five in this case, the gland exhibits normal architecture and lipid accumulation appears to be at baseline by day seven. The gross appearance of the ear remains relatively unchanged at all time courses.

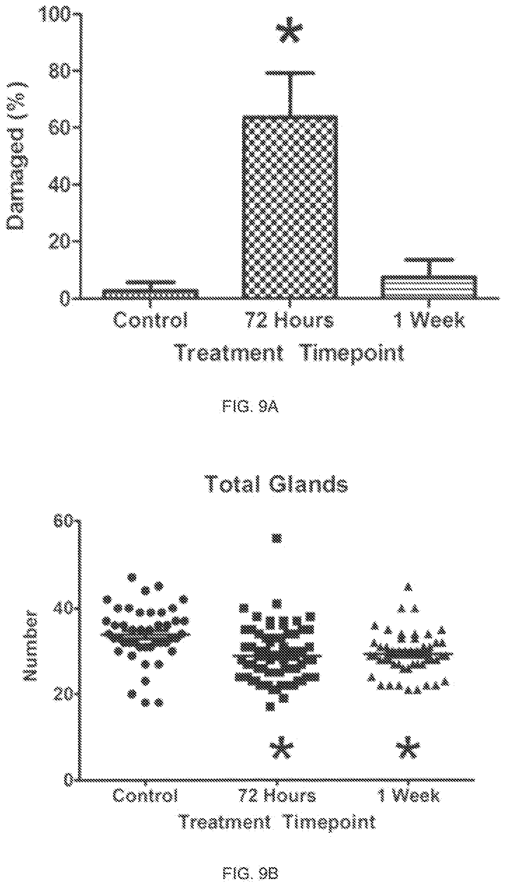

FIGS. 9A and 9B provide graphs illustrating cooling damage of sebaceous glands in a murine model. Glands from sections from three different experiments with the same experimental condition (-7 °C for 10 minutes) were manually counted. Glands were deemed "damaged" if three or more cells necrotic cells were visualized within the sebaceous gland. Over 60% of glands were "damaged" at 72 hours as depicted in FIG. 9A. The total number of glands was decreased at 72 hours and 1 week following treatment as depicted in FIG. 9B. Glands from three different animals treated with the same conditions were evaluated at each time point for each figure. An asterisk (^{∗}) denotes a significant difference (p<0.001) compared to the control group.

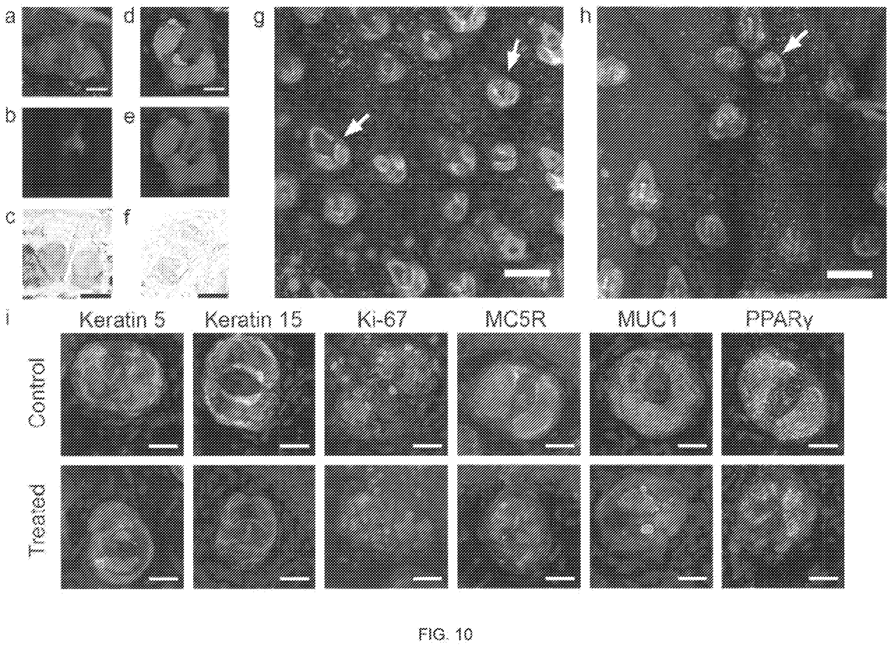

FIG. 10 depicts the disruption of cellular membrane and enzymatic activity and reduced lipid content of sebocytes by cooling. In panels (a), (b), (d), and (e), propidium iodide (PI) staining in treated (panels (d) and (e)) and control glands (panels (a) and (b)) is visible two hours after cooling treatment. Lipids are stained by BODIPY^{®} lipid dye and shown in green. Nuclei are stained by HOECHST^{®} stains and shown in blue. PI is shown in red. As seen in panels (a) and (b), PI staining was minimal in untreated controls and, when present, was restricted to the region immediately adjacent to the hair follicle. Treated glands showed extensive PI staining, which co-localized with both nuclei and sebum lipids as seen in panels (d) and (e). The width of images is 100 m. In panels (c) and (f), alkaline phosphatase was highly active (shown in red) in untreated glands in panel (c), but was substantially diminished in treated glands of panel (f) 3 days after cooling. Panels (g)-(i) depict the reduction of lipid content in sebaceous glands by cooling. Representative z-projection images from 4 different animals are shown. In panels (g) and (h), keratin 5 (red), lipids (green) and nuclei (blue) were labeled by whole-mount staining. In panel (g), untreated sebaceous glands (annotated with white arrows) show expected morphology, namely, basal epithelium expressing keratin 5 and a lipid-filled gland interior. In panel (h), keratin 5 expression was retained in the treated glands 3 days after cooling, but lipid content was substantially reduced, and in some cases almost completely abolished (as annotated with the white arrow). Panel (i) provides high-power views of various protein markers in sebaceous glands labeled by whole mount staining, shown in red. Lipids are shown in green, and nuclei in blue. Cooling did not disrupt the expression of any of these markers, but lipid content was diminished. The scale bars in panels (a)-(f) and (i) represent 25 m. The scale bars in panels (g) and (h) represent 100 m.
FIG. 11 depicts the susceptibility of porcine sebaceous glands cryo-injury through histology of the swine ear. Panel (A) is a control section showing normal sebaceous glands. Panel (B) provides a representative slide from the treatment area 1 week post-treatment, showing both diminished gland density and size. Panel (C) is a representative slide from the treatment area 2 weeks post-treatment showing restoration in gland number but diminished gland size when compared to baseline.
FIG. 12 depicts the reduction of surface area and density of porcine sebaceous glands after cooling. Sebaceous glands on digitized whole slides were manually counted. Gland density was decreased at one week post treatment, when compared to control, and gradually increased at two weeks and four weeks post treatment.
FIG. 13 depicts a cooling curve of representative murine procedure. Arrows represent the start and end of contact time.
FIGS. 14A-14C depict microneedle-based cooling devices 1400 according to the disclosure.

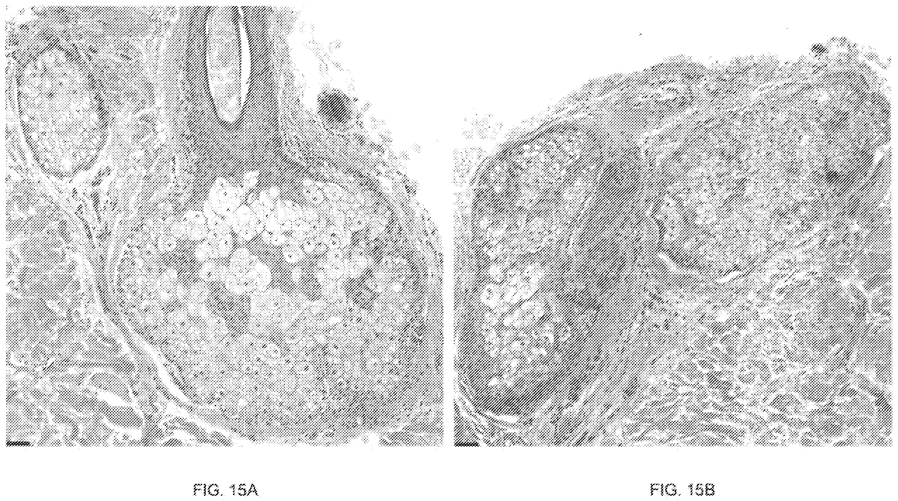

FIGS. 15A and 15B depicts the disruption of cellular architecture in human sebaceous glands. FIG. 15A depicts a sebaceous gland from an untreated area, showing normal sebaceous gland architecture including lipid-laden sebocytes filling the gland interior and sebocytes nearest the gland duct containing increased lipid content and pyknotic nuclei and about to undergo cell lysis as part of the holocrine secretion process. In FIG. 15B, degenerative changes are seen in the sebaceous glands 3 days after cooling at -10° C for 20 minutes, including loss of intracellular lipid granules, pyknotic nuclei throughout the gland body, and the presence of an eosinophilic precipitate. The scale bars in both figures represent 50 m.

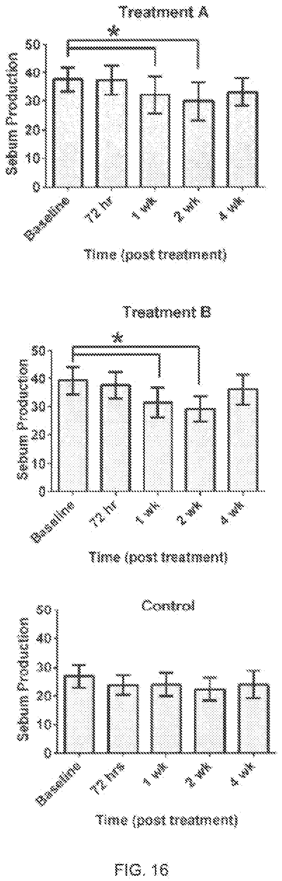

FIG. 16 depicts the suppression of sebum output in humans after cooling. Sebum output was measured at various time points using a SEBUMETER^{™} system. Data from the -15° C and -10° C groups were combined because there was no statistical difference between the two groups. Following cooling treatment of either one 20-minute cycle (Treatment A) or two 10-minute cycles (Treatment B), sebum output was significantly lower than baseline at 1 (p<0.002) and 2 (p<0.0005) weeks post-treatment. In contrast, there was no significant difference in sebum output from untreated sites (Control) across the different time points. Error bars denote ± 95% confidence intervals. An asterisk (^{∗}) denotes a significant difference between post-treatment and baseline sebum output.

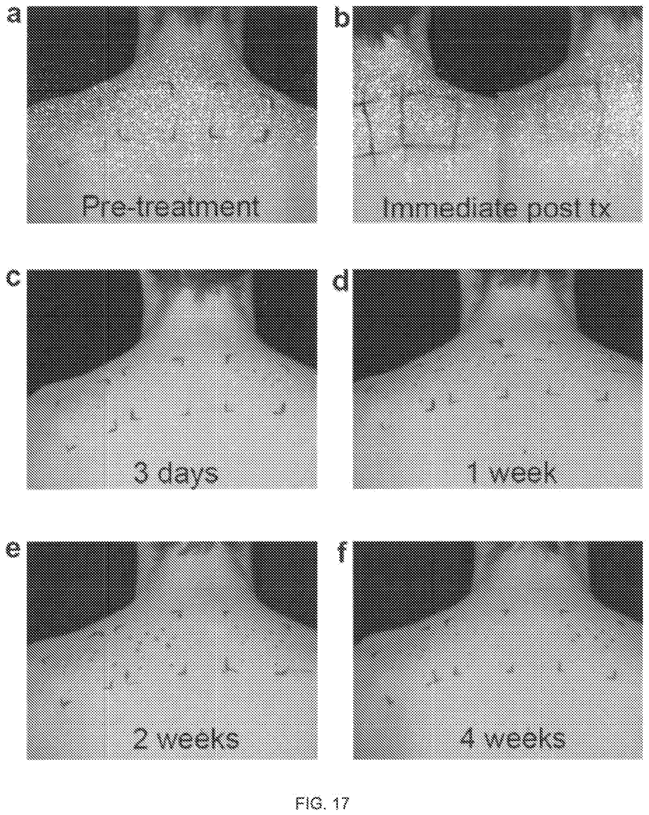

FIG. 17 provides clinical photos from a representative subject taken before and up to 4 weeks after cooling treatment. This particular subject was randomized to the -15 °C treatment. The left lateral site was the untreated control, the left paraspinal site was treated with two separate 10-minute cycles, and the right paraspinal site was treated with one continuous 20-minute cooling cycle. Panel (a) is a photograph taken before the cooling treatment. Panel (b) consists of two photographs taken immediately after the cooling treatment for the left and right paraspinal sites, respectvely. Panels (c), (d), (e), and (f) are photographs taken at 3 days, 1 week, 2 weeks, and 4 weeks, respectively, after the cooling treatment.
FIGS. 18A-18C depict various fractional cooling configurations according to embodiments of the invention.
FIG. 19 depicts a one-dimensional heat transfer model utilized for modeling temperatures produced by cooling a subject's skin.

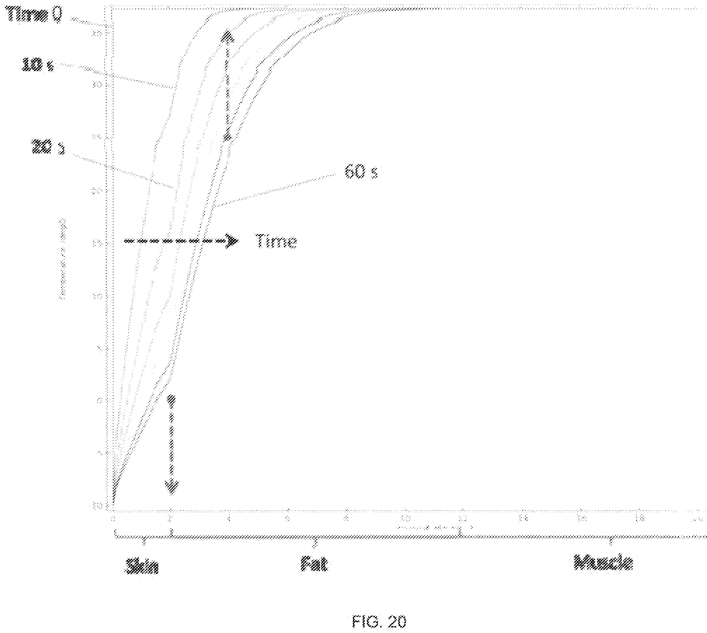

FIGS. 20, 21, and 22 depicts a mathematical model of temperature profiles in tissue in response to cooling with a cooling element at -10° C, -20° C, and -30° C, respecively. Temperature (° C) is plotted on the *x* axis and tissue depth (in mm) is plotted on the y axis. The different curves (colored in the original application) represent temperature profiles at different times in 10 second increments from time 0 on the left to 60 seconds on the right. The dashed arrow pointing up (red in the application as originally filed) at a depth of 4 mm represents 20% of the typical subcutaneous fat thickness. The dashed arrow pointing down (green in the application as originally filed) at a depth of 2 mm represents the typical boundary between the skin and the subcutaneous fat.
FIGS. 23A and 23B depict cross-sections of cooling elements having flat and corrugated cooling surfaces, respectively.

### DEFINITIONS

The instant invention is most clearly understood with reference to the following definitions.

As used in the specification and claims, the singular form "a," "an," and "the" include plural references unless the context clearly dictates otherwise.

Unless specifically stated or obvious from context, as used herein, the term "about" is understood as within a range of normal tolerance in the art, for example within 2 standard deviations of the mean. About can be understood as within 10%, 9%, 8%, 7%, 6%, 5%, 4%, 3%, 2%, 1 %, 0.5%, 0.1 %, 0.05%, or 0.01 % of the stated value. Unless otherwise clear from context, all numerical values provided herein are modified by the term about.

As used in the specification and claims, the terms "comprises," "comprising," "containing," "having," and the like can have the meaning ascribed to them in U.S. patent law and can mean "includes," "including," and the like.

Unless specifically stated or obvious from context, as used herein, the term "or" is understood to be inclusive.

The term "permanent" (and related words such as "permanently") refers to changes in a subject's epidermis that will not return to a previous state over time. Examples of permanent injury to epidermal tissue include scarring and ulceration. Ranges provided herein are understood to be shorthand for all of the values within the range. For example, a range of 1 to 50 is understood to include any number, combination of numbers, or sub-range from the group consisting 1 , 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 (as well as fractions thereof unless the context clearly dictates otherwise).

The term "reversible" (and related words such as "reversibly") refers to changes in a subject's epidermis that will return to a previous state over time. In some embodiments, such reversible changes will return their original state without the need for any medical intervention. Suitable periods of time over which the reversal can occur include less than 1 hour, less than 1 day, less than 1 week, less than 1 month, and the like.

The term "sebaceous gland disorder" is intended to include any condition caused by, exacerbated by, or associated with abnormally high sebum production by sebaceous glands within a local region. Examples of such sebaceous gland disorders include, but are not limited to, *acne vulgaris, nevus sebaceous,* sebaceous gland hyperplasia, and *acne rosacea.*

A "subject" shall be understood to include any mammal including, but not limited to, humans. The term "subject" can include all subsets of individuals within a particular class of subjects, e.g., males, females, infants, children, adolescents, adults, male adolescents, female adolescents, male adults, female adults, and the like.

### DETAILED DESCRIPTION

Aspects of the invention provide cooling devices for at least temporarily disrupting function of one or more sebaceous glands.

Sebaceous glands contain 30% lipid content including triglycerides, wax esters, and squalene. Human sebum reportedly freezes at 15° C *ex vivo.* Aspects of this invention exploit these properties to selectively target and disrupt function of sebaceous glands. This disruption can be temporary (e.g., lasting for a period of hours, days, weeks, months, or years) or can be permanent.

### Methods of At Least Temporarily Disrupting Sebaceous Glands

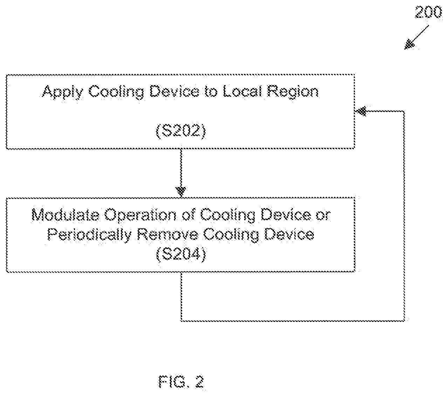

Referring now to FIG. 2, a method 200 of disrupting sebaceous glands is provided, wherein the method is not a part of the invention. The method 200 includes applying a cooling device to skin surface 115 of a local region of a subject (S202) and modulating operation of the cooling device or periodically removing the cooling device in order to cool one or more sebaceous glands 130 within the local region to a temperature sufficient to at least temporarily disrupt function of the one or more sebaceous glands 130 (S204).

Method 200 can utilize any cooling device capable of achieving a sufficient cooling of one or more sebaceous glands within a local region. Without being bound by theory, it is believed that freezing of one or more types of cells within the sebaceous glands 130 retard cell growth and/or cause apoptosis of one or more types of cells within the one or more sebaceous glands 130. Suitable temperatures are believed to be between about 15° C and about 10° C, between about 10° C and about 5° C, between about 5° C and about 0° C, between about 0° C and about -5° C, between about -5° C and about -10° C, between about -10° C and about -15° C, between about -15° C and about -20° C, between about -20° C and about -25° C, and between about -25° C and about -30° C.

As discussed in further detail herein, suitable cooling devices can include topical cooling devices that be held against the epidermis of a local region. The cooling device can be held substantially stationary over a local region or can be translated over the subject's epidermis in order to affect sebaceous glands within a plurality of local regions and facilitate multiple cooling-warming cycles.

### Cooling Devices

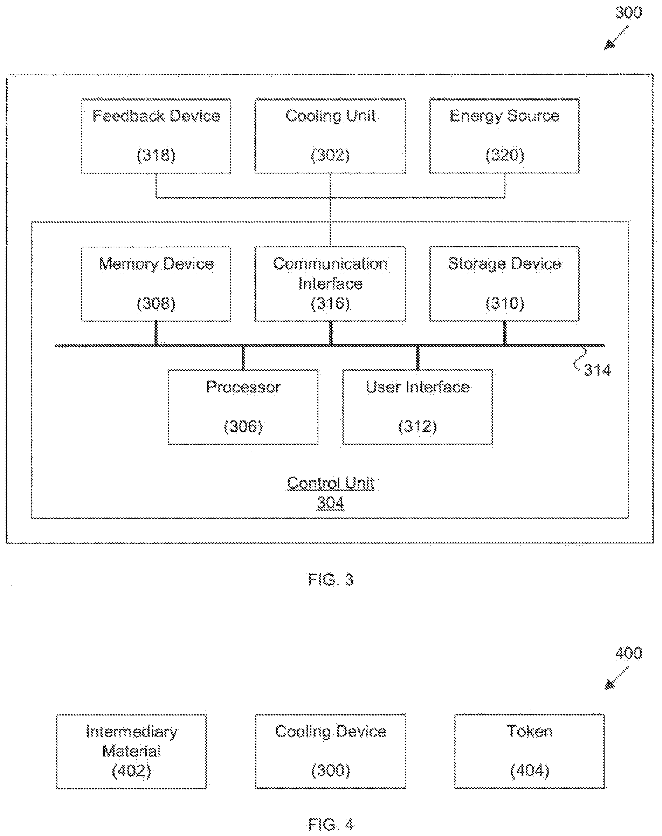

Referring now to FIG. 3, a cooling device 300 can include a cooling unit 302 and a control unit 304.

The cooling unit 302 can be any device capable of achieving a sufficient cooling of one or more sebaceous glands within a local region. Examples of suitable cooling units 302 include thermoelectric (Peltier) coolers, adiabatic cooling devices, fluid-cooled units that communicate with an external heat exchanger, and cryogenic devices that utilize cooled gases such as nitrogen or carbon dioxide to produce the desired low temperatures. Examples of such devices are provided in U.S. Patent Application Publication Nos. 2005/0251120 and 2007/0010861 and in Pascal Laugier et al., "In Vivo Results with a New Device for Ultrasonic Monitoring of Pig Skin Cryosurgery: The Echographic Cryoprobe," 111(2) J. Inves. Derm. 314-19 (1998). In some embodiments, the cooling unit 302 can include one or more nozzles to facilitate a controlled spray of compressed fluid onto the subject's skin. The safety of such a structure can be enhanced by inclusion of physical and/or control structures adapted and configured to ensure that the cooling unit 302 is an appropriate distance from the subject's skin in order to prevent aerosol burns and/or inappropriate discharge of the compressed fluid.

In some embodiments, the cooling unit 302 contains a material that undergoes a phase change (*e.g.,* solid to liquid, solid to gas, liquid to gas, and the like) at a temperature at or near a desired cooling temperature. For example, Sonoco ThermoSafe of Arlington Heights, Illinois produces gels having a phase transition at a specified temperature point (*e.g.,* at -23° C for Model #418).

In some embodiments, the cooling unit 302 can be pre-cooled to a temperature below a desired epidermal temperature. For example, if the cooling unit 302 is cooled to 10° C lower than the desired dwell temperature of the epidermis, a faster initial cooling rate can be achieved without permanently and/or reversibly harming the epidermis because the cooling unit 302 temperature will quickly increase upon contacting the skin.

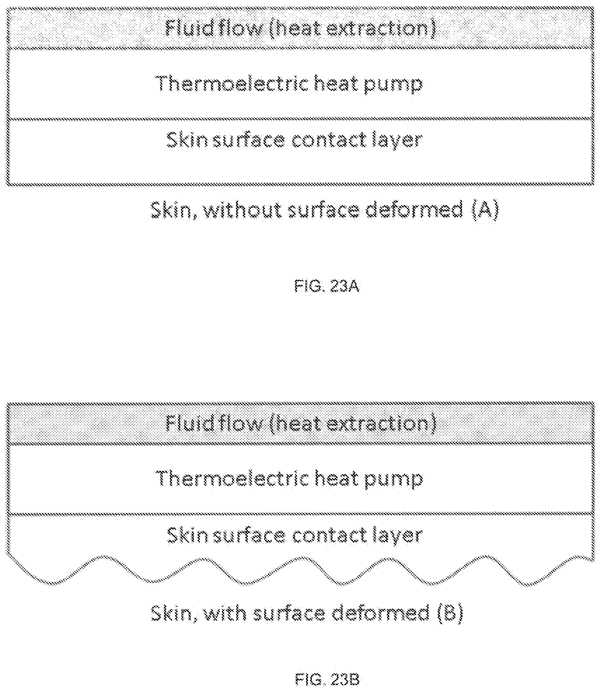

Cooling unit(s) 302 can have a variety of geometries as discussed in U.S. Patent Application Publication Nos. 2005/0251120 and 2007/0010861. In some embodiments, the cooling unit has a contact or cooling surface that is substantially flat, convex, or concave. In other embodiments, the contact or cooling surface has a patterned, dimpled, or corrugated surface to increase contact area and heat transfer with the subject's skin. Cross-sectional views of cooling elements having flat and corrugated cooling surfaces, respectively, are depicted in FIGS. 23A and 23B. In both FIGS. 23A and 23B, a thermoelectric heat pump (*e.g.,* a Peltier element) is used to remove heat from the cooling surface in contact with the skin and optionally output heat to cooling fluid on the opposite side of the thermoelectric heat pump (which can optionally be circulated to an additional thermoelectric heat pump, cooling device, heat sink, or heat exchanger for cooling and recirculation.

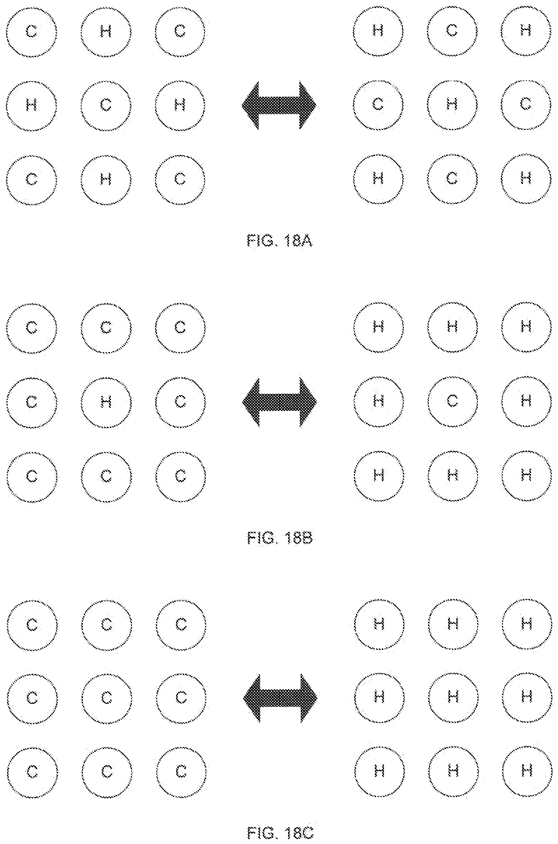

Additionally or alternatively, the cooling unit(s) 302 can be fractional cooling devices having a plurality of posts or other geometries that contact the epidermis to cause a plurality of cooled regions. Fractional cooling devices are described in U.S. Patent Application Publication Nos. 2010/0036295, 2011/0313411, and 2013/0066237 and International Publication No. WO 2013/075006. A two-dimensional array of fractional cooling elements can simultaneously heat and cool adjacent regions and can optionally oscillate between heating and cooling of particularly posts as depicted in FIGS. 18A-18C, utilize translation of the cooling unit(s) 302 across the epidermis, or remain in a fixed location.

Cooling units 302 of the present invention can contain cooling agents in the form of a solid, liquid or gas. Solid cooling agents can comprise, for example thermal conductive materials, such as metals, metal plates, glasses, gels and ice or ice slurries. Liquid cooling agents can comprise, for example, saline, glycerol, alcohol, water/alcohol mixtures, liquid nitrogen, and the like. Where the cooling element includes a circulating cooling agent, preferably the temperature of the cooling agent is constant. Salts can be combined with liquid mixtures to obtain desired temperatures. Gasses can include, for example, cold air, compressed air, and the like.

Control unit 304 can be an electronic device programmed to control the operation of the cooling unit 302 to achieve a desired result. The control unit 304 can be programmed to autonomously carry out a cooling regimen without the need for input (either from feedback devices or medical professionals) or can incorporate such inputs. The principles of how to use feedback (*e.g.,* from a temperature sensor) in order to modulate operation of a component are described, for example, in Karl Johan Åström & Richard M. Murray, Feedback Systems: An Introduction for Scientists & Engineers (2008).

Control unit 304 can be a computing device such as a microcontroller (*e.g*., available under the ARDUINO^{®} OR IOIO^{™} trademarks), general purpose computer (*e.g.,* a personal computer or PC), workstation, mainframe computer system, and so forth. Control unit 304 can include a processor device (*e.g.,* a central processing unit or "CPU") 306, a memory device 308, a storage device 310, a user interface 312, a system bus 314, and a communication interface 316.

Processor 306 can be any type of processing device for carrying out instructions, processing data, and so forth.

Memory device 308 can be any type of memory device including any one or more of random access memory ("RAM"), read-only memory ("ROM"), Flash memory, Electrically Erasable Programmable Read Only Memory ("EEPROM"), and so forth.

Storage device 310 can be any data storage device for reading/writing from/to any removable and/or integrated optical, magnetic, and/or optical-magneto storage medium, and the like (*e.g.,* a hard disk, a compact disc-read-only memory "CD-ROM", CD-ReWritable "CD-RW", Digital Versatile Disc-ROM "DVD-ROM", DVD-RW, and so forth). Storage device 310 can also include a controller/interface for connecting to system bus 314. Thus, memory device 308 and storage device 310 are suitable for storing data as well as instructions for programmed processes for execution on processor 306.

User interface 312 can include a touch screen, control panel, keyboard, keypad, display or any other type of interface, which can be connected to system bus 314 through a corresponding input/output device interface/adapter.

Communication interface 316 can be adapted and configured to communicate with any type of external device, including cooling unit 302. Communication interface 316 can further be adapted and configured to communicate with any system or network, such as one or more computing devices on a local area network ("LAN"), wide area network ("WAN"), the Internet, and so forth. Communication interface 316 can be connected directly to system bus 314 or can be connected through a suitable interface.

Control unit 304 can, thus, provide for executing processes, by itself and/or in cooperation with one or more additional devices, that can include algorithms for controlling cooling unit 302 in accordance with the present invention. Control unit 304 can be programmed or instructed to perform these processes according to any communication protocol and/or programming language on any platform. Thus, the processes can be embodied in data as well as instructions stored in memory device 308 and/or storage device 310 or received at user interface 312 and/or communication interface 316 for execution on processor 306.

Control unit 304 can control the operation of the cooling unit 302 in a variety of ways. For example, control unit 304 can modulate the level of electricity or cooling liquid provided to the cooling unit 302. Alternatively, the control unit 304 can transmit instructions and/or parameters to the cooling unit 302 for implementation by the cooling unit 302.

In addition or as an alternative to modulating the temperature of the cooling unit 302, the control unit 304 can initiate other mechanisms to prevent thermal damage to non-targeted structures. For example, the control unit 304 can initiate an alarm or alert that the cooling unit 302 should be moved to a new location or removed from the subject's skin. In another example, the control unit 304 can control a mechanical, electrical, or electromechanical device to either withdraw a cooling element within a housing of the cooling unit 302 or deploy a mechanical device to lift the cooling unit away from the subject's skin.

Control unit 304 can interface with one or more feedback devices 318 in order to monitor the temperature of one or more components of the subject's skin. A variety of suitable feedback devices are described in U.S. Patent Application Publication Nos. 2005/0251120 and 2007/0010861.

Cooling device 300 can also include one or more energy sources 522. Energy sources 522 can be used to heat particular regions of the subject's skin during operation of the cooling unit 202 and/or to warm one or more sebaceous glands in between cooling cycles.

Suitable energy sources 522 includes radiofrequency (RF) energy generating units that can be adapted, configured, and/or programmed to generate monopolar, bipolar, capacitively-coupled, and/or conductively-coupled RF energy. The RF energy can have a frequency between about 0.3 MHz and about 100 MHz.

Other suitable energy sources 522 include coherent light sources, incoherent light sources, heated fluid sources, resistive (Ohmic) heaters, microwave generators (e.g., producing frequencies between about 915 MHz and about 2.45 GHz), and ultrasound generators (e.g., producing frequencies between about 300 KHZ and about 3 GHz).

### Kits and Consumable Products

Referring now to FIG. 4, cooling device 300 can be provided as part of a larger kit 400 in order to achieve the desired cooling of sebaceous glands 130.

Kit 400 can include one or more consumable products such as an intermediary material 402. Intermediary material 402 can advantageously enhance thermal conductivity between the cooling device 300 and the local region. Additionally or alternatively, intermediary material 402 can be a cryoprotectant that protects the epidermis from permanent and/or reversible damage while the sebaceous glands 130 are cooled.

Intermediary material 402 can be a liquid or a gel. Suitable intermediary materials 402 include water, heavy water, oil, peanut oil, glycerol, glycol, polypropylene glycol (PPG), polyethylene glycol (PEG), propylene glycol, ethylene glycol, dimethyl sulfoxide (DMSO), alcohol, ethanol, propanol, iso-propanol, carboxyl polyethylene polymer, hydroxyethyl xylose polymer, carboxyl methylcellulose, hydroxyethyl cellulose (HEC), the like, and combinations thereof.

Suitable intermediary materials 402 are available from ZELTIQ Aesthetics, Inc. of Pleasanton, California and described in U.S. Patent Application Publication Nos. 2007/255362, 2010/0280582, and 2011/0300079 and U.S. Patent No. 6,041,787.

Intermediary materials 402 can be provided in a variety of formats. Intermediary material 402 can be provided in a dispenser (e.g., a squeeze bottle) for direct application to the subject's epidermis prior to application of the cooling device 300. In another embodiment, intermediary material 302 is impregnated within a pad, textile, foam, sponge, or other porous surface that can be placed on the subject's epidermis prior to application of the cooling device 300. In still another embodiment, the intermediary material 402 is hermetically sealed in a pouch as described for example, in U.S. Patent Application Publication No. 2007/0270925.

System 400 can also include one or more tokens 404 that can be enable licensing and monetization of the systems, methods, and kits on a per use basis. Exemplary tokens 404 are described in U.S. Patent Application Publication No. 2010/0280582.

### Dual-Modality Devices

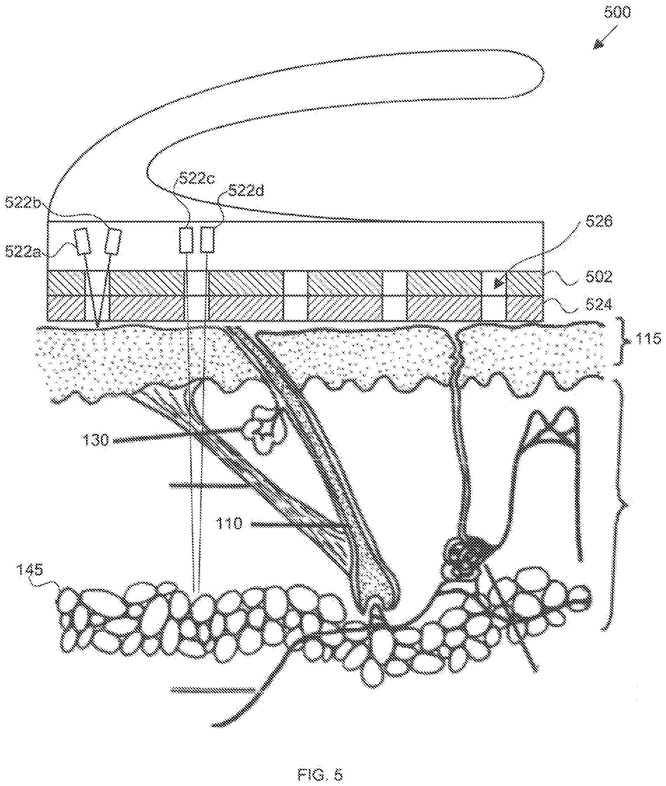

Referring now to FIG. 5, some embodiments of the invention utilize one or more energy sources 522 in order to introduce energy to one or more locations and/or layers of the local region. Such energy can protect particular locations and/or layers from cold-induced damage while one or more sebaceous glands are cooled to a desired temperature.

The device 500 depicted in FIG. 5 includes a plurality of energy sources 522a-522d that are adapted, configured, and/or programmed to direct energy to a particular location at a particular depth within the local region. For example, energy sources 522a-522d can produce a coherent energy beam such as a laser beam. Energy sources 522 can be positioned in order to achieve a desired angle of the beam with respect to a normal to the skin. As seen in FIG. 4, larger angles with respect to normal such as in the context of energy sources 522a, 522b will generally focus the energy on a shallower layer of the local region such as the epidermis 115. Smaller angles with respect to normal such as in the context of energy sources 522c, 522d will generally focus the energy on deeper layers of the local region such as subcutaneous adipose tissue 145.

In some embodiments, energy sources 522 are positioned with a sufficiently high resolution such that a subset of energy sources 522 can be selectively modulated depending on their location relative to a sebaceous gland 130. For example, the local region can be imaged to identify the location of hair follicles 110, adjacent to which sebaceous glands will be present. Energy sources 522 proximate to the hair follicle 110 can be deactivated or modulated to emit less energy relative to energy sources distal to the hair follicle 110 so that the energy sources 522 proximate to the sebaceous gland 130 do not interfere with cooling.

Cooling unit 502 and/or cooling interface 524 can include a plurality of openings 526 in order to permit energy from energy sources 522 to exit device 500 and reach the local region. In some embodiments, cooling unit 502 and/or cooling interface 504 is made from a material that is substantially transparent with respect to the wavelength of energy applied, thereby rendering holes in cooling unit 502 and/or cooling interface 524 unnecessary.

### Cooling Profiles

Control unit 304 can be programmed to implement various cooling algorithms in order to selectively disrupt sebaceous gland function.

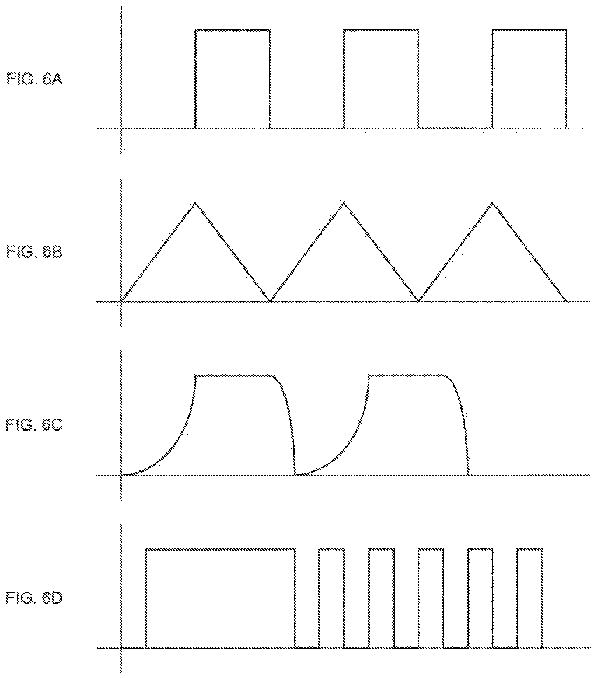

Various cooling profiles are depicted in FIGS. 6A-6D, wherein cooling intensity is shown on the y axis and time is shown on the *x* axis.

The rates of cooling and rewarming can be controlled and/or modulated in order to achieve desired cooling of the sebaceous glands. For example, a defined number of joules per unit of area or volume per unit of time can be extracted.

Without being bound by theory, it is believed that aggressive cooling regimes achieve are most disruptive to sebaceous glands 130. For this reason, it may be preferred to apply a pre-cooled cooling unit (*e.g.,* at a temperature of about -7° C, about -8° C, about -9° C, about -10° C, and the like) to the skin.

Using another example, control unit 304 can actuate cooling unit 302 to cool the local region until the sebaceous glands within the local region reach a desired temperature (e.g., about -7° C or lower). Once this temperature is achieved, the control unit 304 can reduce the cooling power of the cooling unit 302 in order to hold the sebaceous glands at a substantially steady temperature (e.g., about -7° C or lower). Alternatively, control unit 304, can utilize a more discrete method of control in which cooling unit 302 is repeatedly cycled between on and off in order to maintain the substantially steady temperature (e.g., about -7° C or lower).

As discussed above, control unit 304 can receive inputs from one or more feedback devices 318. For example, control unit 304 can control the operation of the cooling unit 302 based on temperature sensors in the cooling unit 302, on a surface of the cooling unit 302, in a treatment interface 524, on a surface of a treatment interface 524, on the surface of a subject's skin 115, or within the subject's skin.

In one embodiment, the control unit 304 can be programmed to monitor for increases in temperature and/or increases in cooling activity by cooling unit 302 (which may be manifested in increased power or coolant draw). Such increases in temperature can be associated, for example, with the latent heat of crystallization one or more portions of the skin freezes as discussed further in U.S. Patent Application Publication No. 2009/0149929. Depending on the degree of the temperature increase and the desirability or undesirability of freezing a particular portion of the skin, cooling can be maintained, increased, decreased, or suspended upon detecting a temperature increase.

In still another embodiment, freeze events can be detected by monitoring for changes in optical properties (*e.g*., scattering, reflection, transmission, coherence, color, and the like) using one or more optical probes (*e.g.,* an optical coherence tomographic device, an optical spectropic device, an IR-reflectoscopic device, a diffuse optical tomographic device, and the like), one or more electrical properties (*e.g.*, resistance, impedance, and the like), and/or one or more acoustic properties (*e.g.*, acoustic velocity, ultrasound acoustic velocity, impedance, and the like) using one or more acoustic probes (*e.g.,* an ultrasound probe, an echographic cryoprobe, and the like) as described in U.S. Patent Application Publication Nos. 2005/0251120 and 2007/0010861.

### Microneedle-Based Cooling

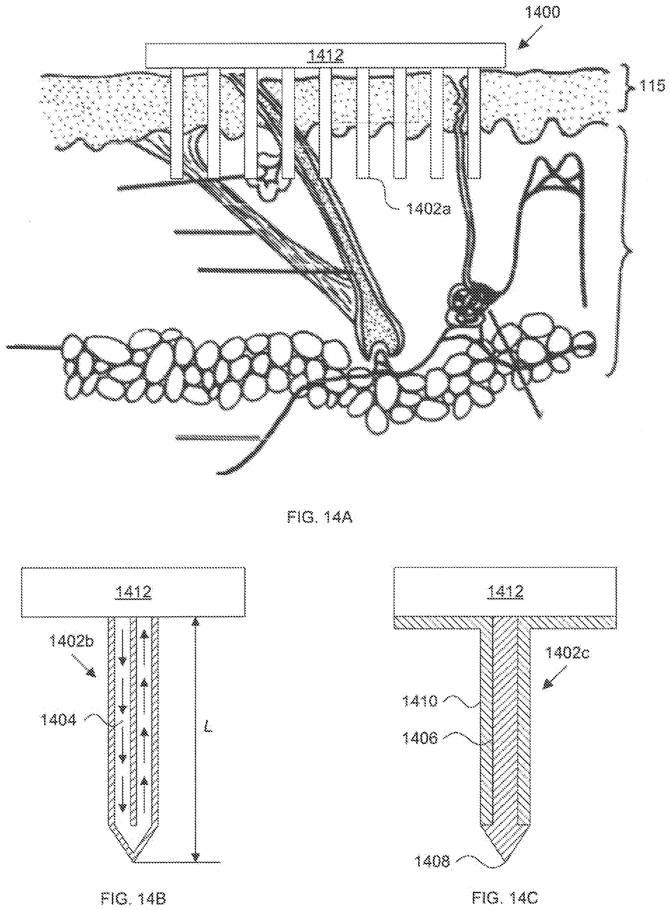

Referring now to FIG. 14A, another aspect of the invention provides a cooling device 1400 including a plurality of microneedles 1402 adapted to penetrate the epidermis 115. Microneedles 1402 can have a working length L adapted to reach the depth of sebaceous glands 130 (typically about 1 mm deep).

Microneedles 1402 can be adapted and configured to apply cooling in the immediate vicinity of the needle, and preferably in the immediate vicinity of the tip of the needle. Various architectures to promote cooling proximate to the microneedle tip are depicted in FIGS. 14B and 14C.

In FIG. 14B, microneedle 1402b includes internal channels 1404 that allow for the recirculating flow of a cooling fluid within the microneedle.

In FIG. 14C, microneedle 1402c has a solid body 1406 with a beveled tip 1408. An insulating layer 1410 surrounds at least a portion of the microneedle shaft in order the minimize cooling of the epidermis. Insulating layer 1410 can optionally extend onto cooling block 1412 in order to minimize cooling of epidermis 115 when microneedles 1402 are fully inserted. Suitable materials for insulating layer 1410 include polymers that are biocompatible, have low thermal conductivity, and/or have low coefficients of friction. Microneedles 1402 preferably have high thermal conductivity in order to quickly cool the layer of the skin containing the sebaceous gland 130.

Microneedles 1402 can be cooled using any cooling means described herein including thermoelectric coolers, cooling liquids, and the like.

Microneedles 1402 can be advanced through the epidermis by pressing cooling device 1400 against the skin. Alternatively, microneedles 1402 can be mechanically, electrically, magnetically, or fluidically extended and retracted, for example, by operation of control unit.

Microneedles 1402 can have a variety of dimensions. For example, microneedles 1402 can have a maximum cross-section of about 160 m, about 245 m, about 465 m, and the like.

Suitable microneedles are described in U.S. Patent Application Publication Nos. 2008/0200910, 2012/0265278, 2013/0184696, 2013/0190745, and 2013/0324990.

### Protection of Epidermal Tissue and/or Subcutaneous Adipose Tissue

In some embodiments, the methods and devices can be controlled in order to produce the desired cooling of one or more sebaceous glands without permanently and/or reversibly injuring epidermal tissue or subcutaneous adipose tissue. The absence of permanent and/or reversible injury to epidermal tissue or subcutaneous adipose tissue can be assessed or verified through a variety of approaches prior to, during, or after development of a particular, method, device, or kit. For example, the absence of epidermal damage can be assessed by visual examination of the epidermis for blistering 48 hours after treatment. The absence of injury to the subcutaneous adipose tissue can be assessed visually by detecting the presence or absence of indentation or contouring seen in U.S. Patent No. 8,840,608 when cryolipolysis is performed, biopsy, ultrasound imaging, and the like. The absence of injury to subcutaneous adipose tissue can also be assessed quantitatively, for example, by assessing whether a certain percentage of adipose tissue (either numerically, dimensionally, or volumetrically) directly below a cooled region was damaged after a period of time (*e.g.,* one month). For example, if the subcutaneous adipose tissue below the application site is reduced (*e.g.,* in adipose cell count, thickness, or volume) by less than 10%, 9%, 8%, 7%, 6%, 5%, 4%, 3%, 2%, or 1% one month after treatment, the procedure can be deemed to have not injured the subcutaneous adipose tissue.

In some embodiments, optimal time and temperature combinations can be augmented as needed to avoid injury to the subcutaneous adipose tissue, for example, by employing periods of passive rewarming, active rewarming, and/or control of cooling rates.

### Thermal Modeling

Temperature profiles in skin, subcutaneous fat, and muscle at the skin surface, were estimated using a 1-dimensional finite element model. Changes in temperature in response to cooling at the skin surface to various target temperatures and durations were simulated, with the goal of delivering sufficient cooling (at or below 0° C) in the skin (particularly the sebaceous glands), while maintaining the subcutaneous fat at a temperature above 25° C. Other thresholds such as maintaining subcutaneous fat above about 20° C, about 15° C, about 10° C, and about 5° C could be utilized as well. Additionally, this modeling assumes for the sake of simplicity that temperature is the only determinant of sebaceous gland disruption, disregarding other factors such as cooling/thawing rates and cooling duration.

Tissue temperature profiles were estimated using a 1-dimensional, time-dependent, finite element model of bioheat transfer, computations were performed using COMSOL MULTIPHYSICS^{®} software (Version 5.0, Comsol AB, Stockholm, Sweden).

The model tissue consisted of four different layers (epidermis, dermis, subcutaneous fat, and muscle), each with the following characteristics specified in Table 1 below.

| Table 1 | | | | | |
|---|---|---|---|---|---|
| | Thickness | Density | Thermal conductivity | Specific heat | Blood perfusion |
| Epidermis | 0.1 mm | 1200 kg/m³ | 0.21 W/m/K | 3600 J/kg/K | 0 kg/m³/s/ blood |
| Dermis | 1.9 mm | 1300 kg/m³ | 0.53 W/m/K | 3800 J/kg/K | 2 kg/m³/s/ blood |
| Subcutaneous fat | 10 mm | 850 kg/m³ | 0.16 W/m/K | 2300 J/kg/K | 0.6 kg/m³/s/ blood |
| Muscle | 10 mm | 1270 kg/m³ | 0.53 W/m/K | 3800 J/kg/K | 0.5 kg/m³/s/ blood |

In addition, the following global parameters applied.

| Table 2 | |
|---|---|
| Initial temperature for all layers | 37° C |
| Specific heat of blood | 4180 J/kg/K |
| Density of blood | 1000 kg/m³ |
| Metabolic heat | 400 W/m³ |

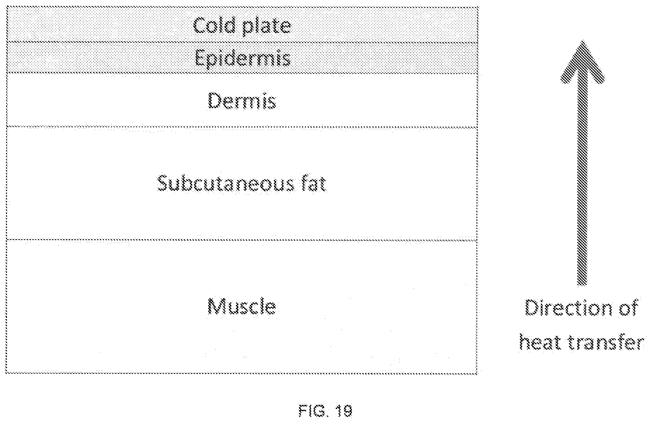

This was a one-dimensional model, *i.e.,* the only concern was with heat transfer in one direction-depth-as depicted in FIG. 19. Heat transfer in the lateral directions was assumed to be negligible. In addition, the cold plate was assumed to be an infinite heat sink operating at constant temperature.

These simulations suggest that cooling with lower temperatures for short periods of time is effective in reducing skin temperatures to sufficient levels to selectively disrupt sebaceous glands, while keeping subcutaneous fat above 25° C.

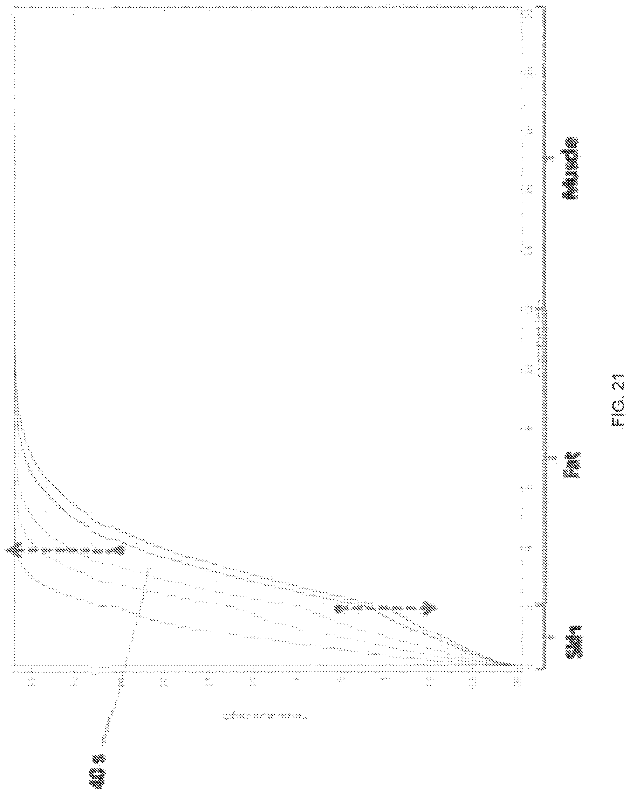

Referring to FIG. 20 and 21, when cooling elements at -10° C or -20° C, respectively, are applied to the skin surface for 10 seconds, the subcutaneous fat (typically beginning 2 mm below the skin surface) can be maintained at or above 25° C (and the subcutaneous fat can be maintained above 5° C during 10-, 20-, 30-, and 40-second applications of a -10° C cooling element and 10- and 20-second applications of a -20° C cooling element).

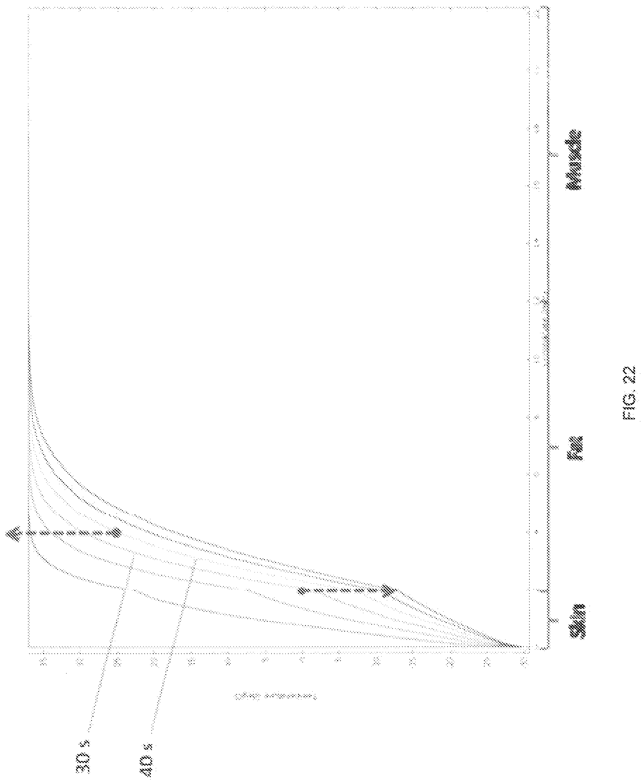

Referring to FIG. 22, when a cooling element at -30° C is applied to the skin surface, the subcutaneous fat can be maintained above 5° C during 10- and 20-second application).

The modeling results indicate that lower cooling temperatures can be combined with short (*e.g.,* less than or equal to 10, 15, or 20 seconds) cooling durations to achieve cooling to target temperature within the skin, while maintaining subcutaneous fat above desired temperatures. If longer total cooling durations and/or repeat freeze-thaw cycles are desired, then a treatment combining short bursts of cooling, each followed by periods of rewarming (*e.g.*, about 2 or 3 times the cooling time) could be used to minimize damage to subcutaneous fat. Additionally, dual-modality devices can provide additional protection to the subcutaneous fat.

In general, sharper temperature profiles are obtained with lower surface cooling temperatures, such that lower temperatures could be achieved within the skin while fat tissue remains relatively warm. Topical cryoprotectants (*e.g.*, glycerol, propylene glycol, and other cryoprotectants described herein) could be applied to at least partially reduce non-specific damage during more aggressive cooling regimens.

### WORKING EXAMPLES

### Materials and Methods

### Animals

Female C57BL/6J mice (16 weeks) were obtained from Jackson Lab (Bar Harbor, Maine). Female Yorkshire (~40 kg) and Yucatan pigs (~ 30 kg) were obtained from Tufts University School of Veterinary Medicine (North Grafton, Massachusetts) and Sinclair BioResources (Windham, Maine), respectively. All animal procedures were performed in compliance with the Public Health Service Policy on Humane Care and Use of Laboratory Animals, and approved by the Massachusetts General Hospital Institutional Animal Care and Use Committee.

### Murine Cooling Experiments

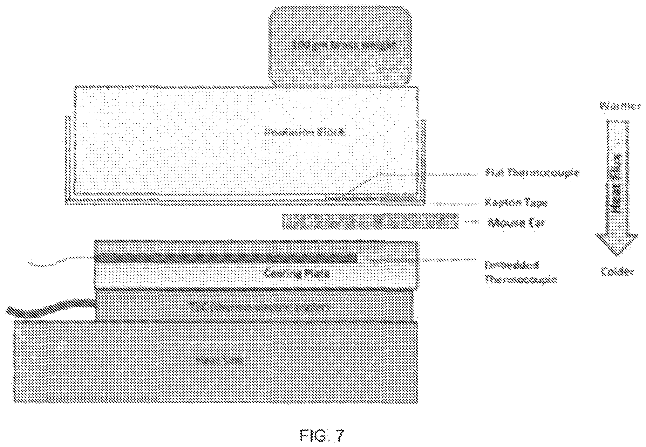

Mice were anesthetized with ketamine-xylazine (90, 9 mg/kg, IP). After anesthesia was obtained, the ear was placed in contact with a cooling plate and held in place with a foam block and a 100-gram weight. The contralateral ear was left untreated for control. A schematic of the cooling equipment and experimental set-up is shown in FIG. 7. Various target temperatures, duration, rates of cooling and re-warming were tested as discussed below. At various time points following the cooling procedure, animals were euthanized and the ear was harvested for histologic analysis. At least 3 independent repeats were performed for each experimental condition.

### Porcine Cooling Experiments

Yorkshire and Yucatan swine were anesthetized with TELAZOL^{®} (tiletamine/zolazepam), xylazine, and atropine (4.4, 2.2, 0.04 mg/kg, IM), followed by maintenance with inhaled isoflurane (1-3%). A thermoelectric cooler measuring 3 cm × 3 cm was placed directly on the ear ridges either with or without the use of a gel pad saturated with propylene glycol (EZ PAD^{™}, Zeltiq Aesthetics, Inc., Pleasanton, California). Ear ridges were chosen for this experiment because they contain a high density of sebaceous glands. The cooling plate was brought to the target temperature and placed onto the ear tissue for 10 minutes. Target temperatures ranged from -5° C to -15° C. A needle thermocouple (Mini-Hypodermic Thermocouple Probe, Model Hyp-0, Omega Engineering, Stamford, Connecticut) was implanted intradermally at a depth of approximately 0.1 mm to monitor tissue temperature. Following cooling, the borders of the treatment area were tattooed. The contralateral ear was left untreated for control. Animals were euthanized at various time points (1 week, 2 weeks, and 4 weeks) and tissue was harvested and processed as described below.

### Clinical Trial

A prospective, site randomized trial was approved by the Institutional Review Board of the Massachusetts General Hospital. Eleven male volunteers between the ages of 18-25 with healthy skin and measurable sebum production were recruited for the study. Subjects with a history of vitiligo, keloid formation, and cold sensitive disorders were excluded from the trial.

Two 2 × 2.25 inch rectangles were marked on either side of the scapula and designated as the treatment sites. Subjects were randomized to one of two treatment temperatures (-10° C and -15° C). Treatment areas on either side of the spine were randomized to receive one 20-minute cycle, or two 10-minute cycles with rewarming up to 10° C surface temperature in between. A site on the lateral scapula was chosen as an untreated control. The treatment sites were marked with surgical markers and photographed before and immediately after treatment, as well as in each subsequent post-treatment time point. Each subject was evaluated before, immediately after, and at 72 hours and 1, 2, and 4 weeks after treatment. At each evaluation, the treatment sites were photographed, sebum production was measured using a SEBUMETER^{™} system available from Courage+Khazaka electronic GmbH of Cologne, Germany following the manufacturer's instructions, and the subjects were questioned regarding symptoms that occurred at the treatment sites. Biopsies where collected 72 hours after treatment and processed for histology, as described in the Histological Processing section below.

### Histological Processing

Tissue was harvested at given time points and fixed in 4% paraformaldehyde in PBS. The tissue was bisected longitudinally and embedded along the long axis. Paraffin-embedded tissues were sectioned in 5 m thickness and stained with hematoxylin and eosin (H&E).

### Quantitative Analysis

For murine sebaceous gland counts, four step sections 100 microns apart from each ear were quantified. Porcine specimens were bisected, embedded cut side down, and step sections were collected 250 microns apart. H&E-stained tissue sections were scanned using a whole mount slide scanner (NANOZOOMER^{®}, Hamamatsu Photonics, Japan). The total number of sebaceous glands in each section was counted by a blinded observer and normalized by the surface length of the corresponding tissue section, and the cross-sectional area of individual sebaceous glands in each section was measured using NDPVIEWER^{™} software (Hamamatsu Photonics). Over 100 sebaceous glands per sample were evaluated for quantitative analysis.

Specific staining for propidium iodide, alkaline phosphatase, lipids, and immunohistochemical markers were performed following previously established protocols described in B.K. Handjinski et al., "Alkaline phosphatase activity and localization during the murine hair cycle," 131 The British Journal of Dermatology 303-10 (1994); I. Martinez-Corral et al., "In vivo imaging of lymphatic vessels in development, wound healing, inflammation, and tumor metastasis," 109 P.N.A.S. 6223-28 (2012); and A. Uemura et al., "Recombinant angiopoietin-1 restores higher-order architecture of growing blood vessels in mice in the absence of mural cells," 110 J. Clin. Invest. 1619-28 (2002).

### Statistical Analysis

In the murine model, repeated measures analysis of variance (ANOVA) was applied to account for multiple measurements per animal in comparing absolute gland number and percentage damaged glands at 72 hours and 1 week compared to control with the Wald test used to test for differences. In addition, a multivariate logistic regression analysis was utilized to evaluate the effects of temperature, rate, duration and cycles of cooling on selective damage of sebaceous glands as a binary outcome. Sebaceous gland size and density were analyzed using ANOVA with the F-test used to compare the effect of treated pig ears at 1, 2, and 4 weeks versus control ears. Statistical analysis was performed using IBM^{®} SPSS^{®} Statistics software (version 21.0, International Business Machines, Armonk, New York). All p-values less than 0.05 were considered statistically significant.

For sebum output measurements in the clinical trial, a mixed-model repeated-measures ANOVA was used in order to account for the two treatment temperatures and the two different treatment durations with longitudinal assessments at 72 hours and 2 weeks. The three sebum measurements for each site per subject were incorporated as replicates in the model. Two-sided p < 0.05 was considered statistically significant with Bonferroni adjustment as appropriate to minimize Type I errors due to multiple comparison. Statistical analysis was performed using IBM^{®} SPSS^{®} Statistics software. P-values less than 0.05 were considered statistically significant.

### Propidium Iodide Staining

Propidium iodide staining was performed following a previously-established protocol discussed in A. Uemura, "Recombinant angiopoietin-1 restores higher-order architecture of growing blood vessels in mice in the absence of mural cells," 110(11) J. Clin. Invest. 1619-28 (2002), with minor modifications. Propidium iodide is a membrane-impermeable dye useful for labeling cells with compromised membranes. Briefly, the mice were given intraperitoneal injections of 400 1 of 1 mg/ml propidium iodide (PI, Sigma-Aldrich, St. Louis, Missouri) 2 hours after cooling treatment (-7° C for 10 minutes), then euthanized after another 2 hours. The ears were collected and immediately frozen in Tissue-Tek O.C.T. compound (Sakura Finetek USA, Torrance, California), and stored at -80° C until use. This procedure was repeated in three animals, with one ear on each animal treated with cooling, while the contralateral ear was left untreated for control. For visualization, 50 m sections of the frozen tissue were prepared using a cryotome, washed, stained with BODIPY^{®} 493/503 lipid dye (1:1000 dilution in phosphate buffered saline, Life Technologies, Grand Island, New York), fixed in 4% formaldehyde for 10 minutes, mounted in PROLONG^{®} GOLD^{™} antifade reagent with DAPI (Life Technologies), and imaged with confocal microscopy.

### Results

### Murine Model

Histologically-selective damage to sebaceous glands, with minimal non-specific injury to surrounding tissues, was observed after exposing mouse ears to appropriate combinations of cooling parameters (Table 3). Each set of experimental conditions was repeated independently on at least 3 animals.

Temperature, cold exposure time, cooling rate, and rewarming rate influenced the outcome. Of the various parameters, one such combination-cooling at -7 °C for 10 minutes-was explored more extensively and is presented below.

In the days following the cooling procedure (-7 °C for 10 minutes), sebaceous glands in the treated regions exhibited a progressive loss of cellular structures, including intracellular lipid granules, nuclei, and intercellular septa, concurrent with accumulation of an eosinophilic precipitate. These histologic signs of cellular damage were first identifiable 2 days after treatment and peaked at 3 days after treatment, presenting in over 60% of the glands. The glands gradually recovered their normal morphology 5-7 days after treatment procedure, and by one week, less than 10% of the glands remained damaged as depicted in FIG. 9A.

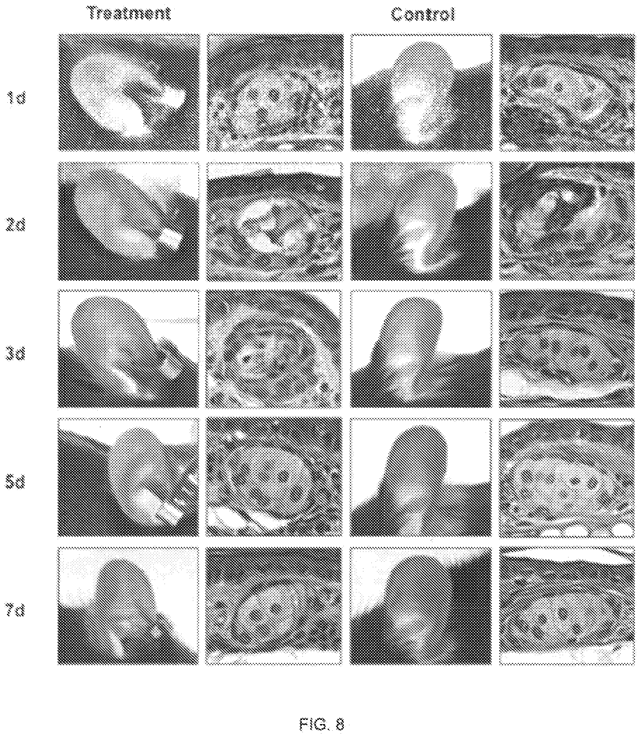

There was transient swelling in the ear after the cooling procedure, but otherwise no gross or histologic evidence of non-specific damage to surrounding skin or ear cartilage tissues were observed (FIG. 8).

The number of sebaceous glands with and without histological evidence for damage was assessed blindly in treated and control ears, in three independent experiments at 72 hours and 1 week after cooling. A gland was deemed as damaged when the aforementioned histologic signs of cellular damage were observed in two or more adjacent sebocytes. Because sebaceous glands secrete sebum via a holocrine mechanism, necrotic cells are normally seen within the glands. In the control specimens, less than 5% of the glands had histologic evidence of damage. At 72 hours post-cooling, an average of 60% of the glands had histologic evidence of damage (p<0.001 compared with controls). At one week, less than 10% of the glands (p<0.001) displayed histologic evidence of cellular damage (FIG. 9A).

Referring now to FIG. 9B, there was a small but significant reduction in the absolute number of glands in the treated samples at 72 hours after cooling (p<0.001). This significant reduction in the number of glands present was sustained at the one week time point despite nearly complete recovery of the histological appearance of the remaining glands (p<0.001), suggesting a prolonged attenuation of gland number due to irreversible damage by cooling.

Referring now to FIG. 10, panels (a), (b), (d), and (e), propidium iodide (PI), a membrane-impermeable dye, was used to label cells with compromised membranes. PI staining was minimal in untreated controls-most glands showed no staining, while a small number of glands had positive staining in the area immediately adjacent to the hair follicle, as depicted in panels (a) and (b), consistent with the normal degeneration of secreted sebocytes. In contrast, post-cooling glands showed extensive propidium iodide (PI) staining throughout the glands, co-localized with both the nuclei and lipids in the glands, as depicted in panels (d) and (e).

Referring now to FIG. 10, panels (c) and (f), alkaline phosphatase activity, normally prominent in sebaceous glands as depicted in panel (c), was substantially diminished as depicted in panel (f) at the time point corresponding to maximal histologic damage (3 days post-cooling). At the same 3-day time point, cooling did not disrupt the expression of key protein markers associated with sebaceous glands as depicted in panels (g)-(i), including keratins 5 and 15, Ki67, MUC1 (Mucin 1, Cell Surface Associated), MC5R (melanocortin receptor-5), and PPAR (Peroxisome Proliferator-Activated Receptor Gamma). However, lipid contents in the treated glands were substantially reduced, and in some cases completely abolished as depicted in panel (h).

### Porcine Model

The porcine ear has gross and micro-scale anatomy that is similar to human sebaceous skin as discussed in F.H. Sakamoto et al., "Porphyrin distribution after topical aminolevulinic acid in a novel porcine model of sebaceous skin," 41 Lasers Surg. Med. 154-60 (2009).

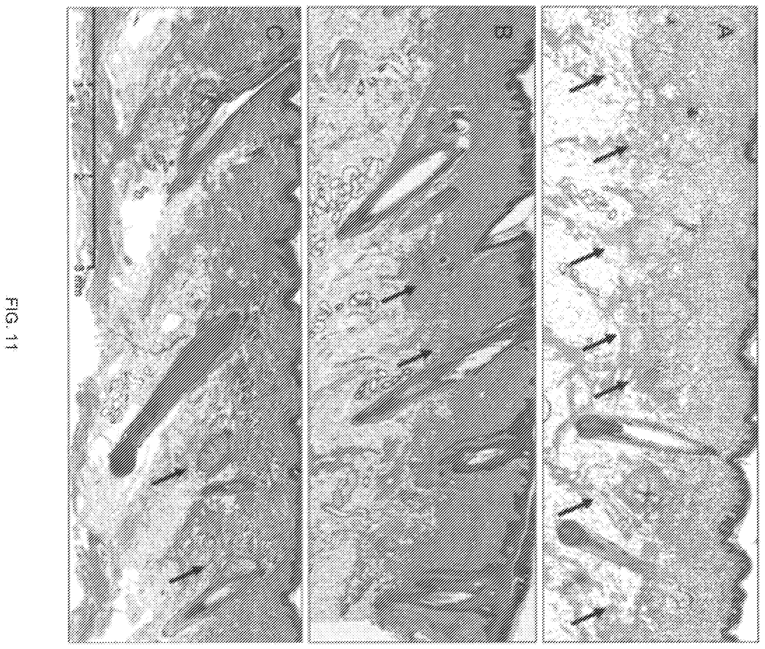

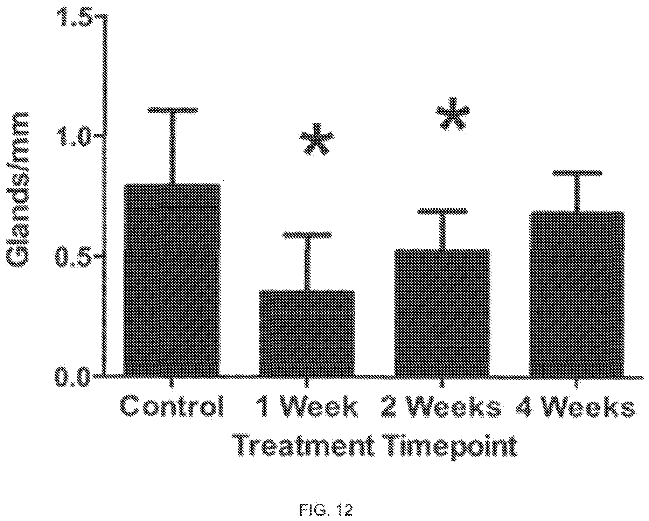

Similar to the murine model, sebaceous glands in the porcine ear model were selectively damaged by a controlled cycle of cooling. One week after cooling at the skin surface, sebaceous glands in the treated areas were markedly decreased in size, and in some treated sites were absent. When present, the sebaceous glands often exhibited loss of cellular structures and the accumulation of eosinophilic precipitates as depicted in FIG. 11, panel (B). At two weeks following treatment, the number of glands had largely returned to normal, however gland size was still decreased as depicted in FIG. 11, panel (C). Gland density, expressed as gland number per mm of skin surface length in cross-section, showed a significant decrease 1 week post-treatment (p<0.001). There was a gradual increase in gland density towards baseline at two weeks post treatment (p<0.005) as depicted in FIG. 12. Desquamation was occasionally observed about 3 days after cooling; otherwise, there were no gross or histological signs of non-specific tissue damage outside of the sebaceous glands.

### Human Study

A pilot clinical trial was conducted in 11 healthy male volunteers with measurable sebum output. Fair-skinned subjects of Fitzpatrick skin phototypes I-III were enrolled, to limit the potential risk of cold-induced hypopigmentation, which was not observed in any subject. Subjects were randomized to one of two cooling device target temperatures (-10° C or -15° C). Two treatment areas of their backs were cooled either for a single 20-minute cycle, or two 10-minute cycles with re-warming in between. Each subject also had one untreated area in the same anatomic region to serve as control. Biopsies taken 72 hours after treatment showed obvious yet selective damage in sebaceous glands, with necrosis and architectural distortion of lipid-laden sebocytes within the sebaceous gland as depicted in FIGS. 15A and 15B. No histologic evidence of non-selective tissue damage was observed. Similar to the murine results, expression of the proliferative marker ki67 and the progenitor basal cell marker keratin 15 was not disrupted by cooling, as determined by immunohistochemistry, which is consistent with the ability of sebaceous glands to eventually recover after treatment. Sebum production decreased significantly at 1 and 2 weeks after treatment, then recovered to baseline levels by week 4 as depicted in FIG. 16. There were no significant differences in any of the measured outcomes between the different treatment temperatures and cycles. The treatment was very well tolerated. There was localized, mild, transient erythema, edema, and dysesthesia after cooling, all of which resolved spontaneously within 72 hours as depicted in FIG. 17. During treatment the subjects reported an average score of 3 on a 10-point Visual Analog Scale for pain, which fell to less than 1 immediately post-treatment, and was 0 by 72 hours.

### Discussion

This study showed that sebaceous glands can be preferentially damaged by controlled cooling at the skin surface, with minimal (in mice) to no (in humans) non-specific injury to surrounding tissues under the conditions studied. Cooling temporarily disrupted cellular architecture, membrane integrity, enzymatic activity, and reduced lipid content in sebaceous glands. In humans, cooling using a -10° C or -15° C device for 20 minutes resulted in an approximate 20% suppression of sebum output that persisted for over 2 weeks.

Temporary histologic damage of murine sebaceous glands with controlled cooling at a variety of temperature and cooling time combinations was achieved. In the porcine model, a transient although significant reduction in sebaceous gland density and surface area was observed for two weeks following cooling and is presented in Table 4.

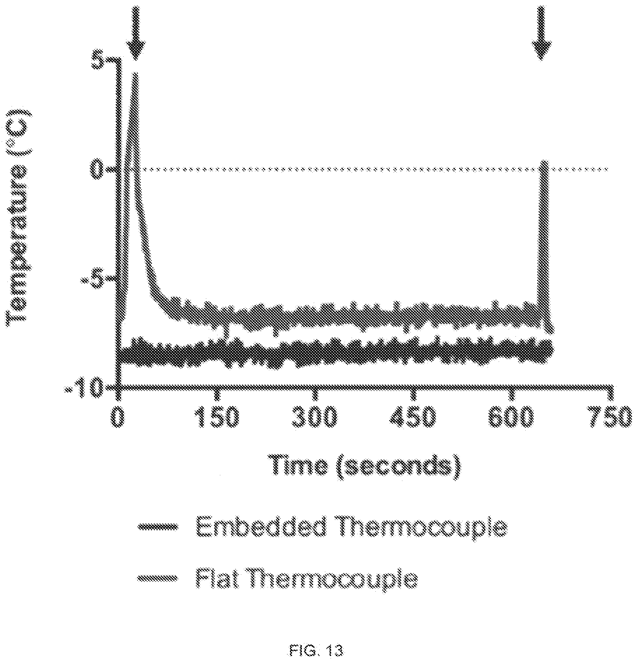

The murine experiments illustrate the relative importance of the cooling parameters of temperature, rate of cooling, and duration of cold exposure. The most important factors to achieve consistent and selective damage to sebaceous glands were the rate of cooling, and the target temperature. In the murine model, there was a ~2° C temperature gradient across the ear, between the cooling plate and the non-contact side of the ear as measured by thermocouples (FIG. 7). The ear temperature and this temperature gradient were stable within 60 seconds of placing the cooling plate on the ear (FIG. 13).

Interestingly, the ear tissue temperature associated with preferential damage to sebaceous glands was far lower than would be predicted based only on the freezing point of human sebum. Without being bound by theory, it is possible that (i) both a "water-freeze" and a "lipid-freeze" contribute to the histologic endpoint and that the relatively low water content of sebocytes may predispose these and similar cells to lethal injury by combined lipid and water crystallization and/or (ii) the different physiologic properties of murine and human sebum, including molecular composition and lipid content, accounts for this difference. For example, human sebum has higher proportions of triglycerides, wax esters, and squalene, but lower proportion of cholesterol when compared to murine sebum.

Increased numbers of freeze-thaw cycles were more destructive, and resulted in non-specific injury to tissue. Slower thawing was also more destructive, and increased non-specific tissue injury.

The porcine ear has gross and micro-scale anatomy that is similar to human sebaceous skin. The mouse data was extrapolated to the porcine model, which revealed reduction in sebaceous gland density after controlled cooling. Using various cooling parameters in both the murine and porcine models, preferential and prompt injury of sebaceous glands was achieved. In mice, there was nearly complete recovery of normal sebaceous gland histology only one week after cooling at -7° C for 10 minutes, but the number of glands was reduced. In swine, which have much larger and deeper sebaceous glands, the glands were smaller even after recovering their normal histological appearance.

The rapid onset of histologic damage following cooling has implications for potential mechanism. Controlled cryoinjury to adipocytes, which leads to intracellular lipid crystallization, induces a pro-apoptotic cascade that results in a delayed panniculitis and fat reduction two to three months following the procedure. The rapid formation of an eosinophilic precipitate within the sebaceous glands in the experiments is similar to what is seen in traditional cryosurgery. *In vitro* data supports the preferential temperature sensitivity of sebocytes and the induction of apoptosis after prolonged cold exposure.

There were few or no side effects and no scarring after controlled cooling that preferentially affected sebaceous glands. Side effects were limited to transient edema in the murine model. In the swine ears at -15° C, desquamation was observed at 72 hours followed by transient hypopigmentation at 1 week. This hypopigmentation resolved with perifollicular repigmentation of the treatment area. There were no gross or histological signs of permanent, non-specific tissue damage outside of the sebaceous glands.

The extensive PI staining that occurred in sebaceous glands shortly (within hours) after cooling treatment indicates that the integrity of the sebocytes' cell and nuclear membranes was compromised by cooling. PI staining was minimal in untreated controls and, when present, was restricted to the area immediately adjacent to the hair follicle, which corresponds to the location where mature sebocytes normally undergo cell death and release their lipid contents as part of the holocrine secretion mechanism. Sebaceous glands normally have a high level of endogenous alkaline phosphatase (ALP) activity. This activity was substantially diminished 3 days after cooling, indicating that the treatment had at least temporarily disrupted some enzymatic machinery of the sebaceous glands. Interestingly, ALP activity remained high in untreated glands, even after prolonged storage at -80° C, which suggests that the post-treatment reduction in ALP activity was not merely a consequence of exposure to low temperatures, but rather a cellular response to the cooling treatment.

Lipid content was also reduced in the treated glands, which is consistent the reduced post-treatment sebum output seen in the clinical trial in FIG. 16.

The co-localization of PI with sebum lipid is an unexpected finding, as PI is generally considered a nuclear stain, although cytoplasmic staining has been reported previously. This atypical staining pattern may be caused by the compromised cell membrane following cooling, which may have simply allowed PI to enter and accumulate within the intracellular lipid depots.

The pilot clinical trial showed the feasibility of selectively damaging human sebaceous glands with controlled cooling, at parameters that were safe and easily tolerated by all eleven subjects in the study. The reduced sebum excretion following a single application of cooling in healthy human subjects strongly suggests that the histologic damage observed correlates with decreased sebum output.

The severity of cooling-induced injury to sebaceous glands achieved in the study appears to be temporary. However, because the complete absence of sebum could potentially have undesirable effects on skin health, the temporary suppression of sebum output may be preferable to permanently destroying sebaceous glands. The sebum reduction in the human study lasted for at least 2 weeks following a single cooling treatment. These results suggest that a treatment interval of about 2 weeks may be able to provide prolonged suppression of sebaceous gland activity.

| Table 3 | | | | | |
|---|---|---|---|---|---|
| Constant | Variable | | | | |
| | Temperature | | | | |
| Duration = 10 minutes | 0° C | -5° C | -7° C | -10° C | |
| | - | ± | + | + | SG |
| Maximal cooling rate | - | - | - | + | NS |

| | Duration | | | | |
|---|---|---|---|---|---|
| Temperature = -7° C | 0 minutes | 5 minutes | 10 minutes | 15 minutes | |
| | - | - | + | + | SG |
| Maximal cooling rate | - | - | - | - | NS |

| | Rate | | | | |
|---|---|---|---|---|---|
| Temperature = -7° C | Cooling Rate | | | | |
| | Max | 2 minutes | Max | 2 minutes | |
| Duration = 10 minutes | Thawing Rate | | | | |
| | Ambient | Ambient | 2 minutes | 2 minutes | |
| | + | - | + | - | SG |
| | - | - | + | - | NS |

| | Freeze-Thaw Cycles | | | | |
|---|---|---|---|---|---|
| Temperature = -7° C | One 10 minute cycle | Two 10 minute cycle | Two 5 minute cycle | Five 2 minute cycle | |
| | + | + | + | + | SG |
| | - | + | + | + | NS |

| | | | | | |
|---|---|---|---|---|---|
| "NS" denotes non-specific injury. "SG" denotes sebaceous gland injury. Plus signs (+) denotes histologic signs of injury. Minus signs (-) denotes no sign of injury. | | | | | |

| Table 4: Porcine Ear Measurements for Control and Treated Ears in Size, Surface Area, and Gland Density | | |
|---|---|---|
| Time | Cross-sectional area (mm²) | Gland Density (number/mm) |
| Control | 0.073 ± 0.082 | 0.79 ± 0.32 |
| 1 Week | 0.016 ± 0.015^{∗} | 0.35 ± 0.24^{∗} |
| 2 Weeks | 0.039 ± 0.041^{∗} | 0.52 ± 0.17^{#} |
| ANOVA | F = 20.6.84, P < 0.001 | F = 19.20, P < 0.001 |

| | | |
|---|---|---|
| Data are mean ± SD. Asterisks (^{∗}) denote P < 0.001 compared to control. Number signs (#) denote P = 0.003 compared to control. | | |

The invention is defined in the following claims. Other embodiments, equivalents, examples and methods do not constitute a part of the invention and are described for illustrative purposes only.

## Claims

1. A cooling device comprising:
a cooling unit; and
a control unit programmed to control operation of the cooling unit in order to cool one or more sebaceous glands within a local region for a period of time and to a temperature below 15° C to disrupt function of the one or more sebaceous glands; and
wherein the control unit is programmed to control operation of the cooling unit to:
cool the one of more sebaceous glands for a first period of time;
**characterised in that** the control unit is further programmed to control operation of
the cooling unit to:
actively or passively warm the one or more sebaceous glands, in order to maintain subcutaneous adipose tissue in the temperature range above 25° C, by one of:
a. actuation of an energy source to introduce energy into the local region;
b. controlling the cooling unit to actively raise the temperature of the cooling unit;
c. suspending or reducing operation of the cooling unit;
d. a plurality of cooling-warming cycles; and cool the one or more sebaceous glands for a second period of time.

2. A cooling device, comprising:
a cooling unit; and
a control unit programmed to control operation of the cooling unit in order to cool one or more sebaceous glands within a local region for a period of time, and to a temperature below 15°C to disrupt function of the one or more sebaceous glands;
**characterised in that** the control unit is programmed to control operation of the cooling unit to employ periods of passive rewarming, active rewarming, and/or control cooling rates so as to maintain subcutaneous adipose tissue in the temperature range above 25°C.

3. The cooling device of claim 1, wherein the energy source is selected from the group consisting of a coherent light source, an incoherent light source, a heated fluid source, a resistive heater, a microwave generator that preferably produces frequencies between about 915 MHz and about 2.45 GHz, and an ultrasound generator that preferably produces frequencies in the range 300 kHz to 3 GHz.

4. The cooling device of any of the previous claims, wherein the control unit is programmed to control operation of the cooling unit to cool the one or more sebaceous glands below a temperature selected from the group consisting of: 14° C, 13° C, 12° C, 11° C, 10° C, 9° C, 8° C, 7° C, 6° C, 5° C, 4° C, 3° C, 2° C, 1° C, 0° C, -1° C, -2° C, -3° C, -4° C, -5° C, -6° C, -7° C, -8° C, -9° C, and -10° C; and/or, wherein the control unit is programmed to control operation of the cooling unit to cool the sebaceous glands at a rate selected from the group consisting of: between 20° C per minute and 30° C per minute, between 30° C per minute and 40° C per minute, between 40° C per minute and 50° C per minute, and between 50° C per minute and 60° C per minute; and/or, wherein the control unit is programmed to control operation of the cooling unit to cool the sebaceous glands at a rate selected from the group consisting of: between 1° C per second and 2° C per second, between 2° C per second and 3° C per second, between 3° C per second and 4° C per second, between 4° C per second and 5° C per second, between 5° C per second and 6° C per second, between 6° C per second and 7° C per second, between 7° C per second and 8° C per second, between 8° C per second and 9° C per second, and between 9° C per second and 10° C per second.

5. The cooling device of any of the previous claims, wherein the period of time, the first period of time, or the second period of time is selected from the group consisting of: less than 10 seconds, between 10 seconds and 20 seconds, between 20 seconds and 30 seconds, between 30 seconds and 40 seconds, between 40 seconds and 1 minute, between 1 minute and 2 minutes, and between 2 minutes and 5 minutes.

6. The cooling device of any of the previous claims, wherein the control unit is programmed to control operation of the energy source to warm the sebaceous glands at a rate selected from the group consisting of: between 20° C per minute and 30° C per minute, between 30° C per minute and 40° C per minute, between 40° C per minute and 50° C per minute, and between 50° C per minute and 60° C per minute; or wherein the control unit is programmed to control operation of the energy source of the cooling unit to warm the sebaceous glands at a rate selected from the group consisting of: between 1° C per second and 2° C per second, between 2° C per second and 3° C per second, between 3° C per second and 4° C per second, between 4° C per second and 5° C per second, between 5° C per second and 6° C per second, between 6° C per second and 7° C per second, between 7° C per second and 8° C per second, between 8° C per second and 9° C per second, and between 9° C per second and 10° C per second.

7. The cooling device of any of the previous claims, wherein the period of time, the first period of time, or the second period of time is selected from the group consisting of: less than 5 minutes, between 5 minutes and 10 minutes, between 10 minutes and 15 minutes, between 15 minutes and 20 minutes, between 20 minutes and 25 minutes, between 25 minutes and 30 minutes, between 30 minutes and 35 minutes, between 35 minutes and 40 minutes, between 40 minutes and 45 minutes, between 45 minutes and 50 minutes, between 50 minutes and 55 minutes, and between 55 minutes and 60 minutes.

8. The cooling device of any of the previous claims, wherein the control unit is programmed to control operation of the cooling unit to cool the one or more sebaceous glands below a temperature selected from the group consisting of: -11° C, -12° C, -13° C, -14° C, -15° C, -16° C, -17° C, -18° C, -19° C, -20° C, -21° C, -22° C, -23° C, -24° C, -25° C, -26° C, -27° C, -28° C, -29° C, and -30° C.

9. The cooling device of any of the previous claims, wherein the cooling device further comprises one or more constriction elements and wherein the control unit is further programmed to:
tighten the one or more constriction elements in order to reduce blood flow to the local region during the cooling steps; and
loosen the one or more constriction elements in order to restore normal blood flow to the local region during the warming step.

10. The cooling device of any of the previous claims, wherein the control unit is programmed to control operation of the cooling unit to subject the one or more sebaceous glands to a plurality of cooling- warming cycles; preferably, wherein the plurality of cooling-warming cycles is selected from the group consisting of: 2, 3, 4, 5, 6, 7, 8, 9, and 10.

11. The cooling device of any of the previous claims, further comprising:
a treatment interface in thermal communication with the cooling unit, the treatment interface adapted and configured for application to the local region, wherein the treatment interface is a mask having a complementary surface that substantially approximates a human face, the treatment interface has a convex surface or a concave surface; preferably, further comprising:
a vacuum in communication with the treatment interface, the vacuum being adapted and configured to generate reduced pressure in order to hold the treatment interface against the local region, and one or more feedback devices in communication with the control unit.

12. A kit comprising:
the cooling device of any of the previous claims;
an intermediary material; and
instructions to apply the intermediary material to the local region prior to application of the cooling device.

13. The kit of claim 12, wherein the intermediary material enhances thermal conductivity between the cooling device, wherein the intermediary material is a cryoprotectant; or, wherein the intermediary material includes one or more selected from the group consisting: water, heavy water, oil, peanut oil, glycerol, glycol, polypropylene glycol (PPG), polyethylene glycol (PEG), propylene glycol, ethylene glycol, dimethyl sulfoxide (DMSO), alcohol, ethanol, propanol, iso-propanol, carboxyl polyethylene polymer, hydroxyethyl xylose polymer, carboxyl methylcellulose, and hydroxyethyl cellulose (HEC).

14. The cooling device as previously defined, comprising energy sources (522a, 522b) configured to focus the energy on the epidermis and/or at the depth of subcutaneous adipose tissue (145).

15. The cooling device as previously defined, comprising energy sources (522a, 522b), wherein the cooling unit (502) includes a plurality of openings (526) in order to permit energy from the energy sources to exit the cooling device (500), or wherein the cooling unit (502) is made from a material that is substantially transparent with respect to the wavelength of energy applied.

## Patentansprüche

1. Kühlvorrichtung, Folgendes umfassend:
eine Kühleinheit; und
eine Steuereinheit, die programmiert ist, um einen Betrieb der Kühleinheit zu steuern, um eine oder mehrere Talgdrüsen innerhalb einer lokalen Region für einen Zeitraum und auf eine Temperatur unter 15 °C zu kühlen, um eine Funktion der einen oder mehreren Talgdrüsen zu unterbrechen; und
wobei die Steuereinheit programmiert ist, um den Betrieb der Kühleinheit für Folgendes zu steuern:
Kühlen der einen oder mehreren Talgdrüsen für einen ersten Zeitraum;
**dadurch gekennzeichnet, dass** die Steuereinheit ferner programmiert ist, um den Betrieb der Kühleinheit für Folgendes zu steuern:
aktives oder passives Erwärmen der einen oder mehreren Talgdrüsen, um Unterhautfettgewebe in dem Temperaturbereich über 25 °C durch Folgendes beizubehalten:
a. Betätigen einer Energiequelle, um Energie in die lokale Region einzuführen;
b. Steuern der Kühleinheit, um die Temperatur der Kühleinheit aktiv zu erhöhen;
c. Unterbrechen oder Reduzieren des Betriebs der Kühleinheit; oder
d. mehrere Kühl-Erwärmungs-Zyklen;
und Kühlen der einen oder mehreren Talgdrüsen für einen zweiten Zeitraum.

2. Kühlvorrichtung, Folgendes umfassend:
eine Kühleinheit; und
eine Steuereinheit, die programmiert ist, um einen Betrieb der Kühleinheit zu steuern, um eine oder mehrere Talgdrüsen innerhalb einer lokalen Region für einen Zeitraum und auf eine Temperatur unter 15 °C zu kühlen, um eine Funktion der einen oder mehreren Talgdrüsen zu unterbrechen;
**dadurch gekennzeichnet, dass** die Steuereinheit programmiert ist, um den Betrieb der Kühleinheit zu steuern, um Perioden einer passiven Wiedererwärmung, einer aktiven Wiedererwärmung anzuwenden und/oder die Kühlgeschwindigkeit zu steuern, um Unterhautfettgewebe in dem Temperaturbereich über 25 °C beizubehalten.

3. Kühlvorrichtung nach Anspruch 1, wobei die Energiequelle aus der Gruppe ausgewählt ist, die aus einer kohärenten Lichtquelle, einer inkohärenten Lichtquelle, einer beheizten Fluidquelle, einem Heizwiderstand, einem Mikrowellengenerator, der vorzugsweise Frequenzen zwischen etwa 915 MHz und etwa 2,45 GHz erzeugt, und einem Ultraschallgenerator besteht, der vorzugsweise Frequenzen in dem Bereich von 300 kHz bis 3 GHz erzeugt.

4. Kühlvorrichtung nach einem der vorhergehenden Ansprüche, wobei die Steuereinheit programmiert ist, um den Betrieb der Kühleinheit zu steuern, um die eine oder mehreren Talgdrüsen unter eine Temperatur zu kühlen, die aus der Gruppe ausgewählt ist, die aus Folgendem besteht: 14 °C, 13 °C, 12 °C, 11 °C, 10 °C, 9 °C, 8 °C, 7 °C, 6 °C, 5 °C, 4 °C, 3 °C, 2 °C, 1 °C, 0 °C, -1 °C, -2 °C, -3 °C, -4 °C, -5 °C, -6 °C, -7 °C, -8 °C, -9 °C und - 10 °C; und/oder wobei die Steuereinheit programmiert ist, um den Betrieb der Kühleinheit zu steuern, um die Talgdrüsen bei einer Geschwindigkeit zu kühlen, die aus der Gruppe ausgewählt ist, die aus Folgendem besteht: zwischen 20 °C pro Minute und 30 °C pro Minute, zwischen 30 °C pro Minute und 40 °C pro Minute, zwischen 40 °C pro Minute und 50 °C pro Minute und zwischen 50 °C pro Minute und 60 °C pro Minute; und/oder wobei die Steuereinheit programmiert ist, um den Betrieb der Kühleinheit zu steuern, um die Talgdrüsen bei einer Geschwindigkeit zu kühlen, die aus der Gruppe ausgewählt ist, die aus Folgendem besteht: zwischen 1 °C pro Sekunde und 2 °C pro Sekunde, zwischen 2 °C pro Sekunde und 3 °C pro Sekunde, zwischen 3 °C pro Sekunde und 4 °C pro Sekunde, zwischen 4 °C pro Sekunde und 5 °C pro Sekunde, zwischen 5 °C pro Sekunde und 6 °C pro Sekunde, zwischen 6 °C pro Sekunde und 7 °C pro Sekunde, zwischen 7 °C pro Sekunde und 8 °C pro Sekunde, zwischen 8 °C pro Sekunde und 9 °C pro Sekunde und zwischen 9 °C pro Sekunde und 10 °C pro Sekunde.

5. Kühlvorrichtung nach einem der vorhergehenden Ansprüche, wobei der Zeitraum, der erste Zeitraum oder der zweite Zeitraum aus der Gruppe ausgewählt ist, die aus Folgendem besteht: weniger als 10 Sekunden, zwischen 10 Sekunden und 20 Sekunden, zwischen 20 Sekunden und 30 Sekunden, zwischen 30 Sekunden und 40 Sekunden, zwischen 40 Sekunden und 1 Minute, zwischen 1 Minute und 2 Minuten und zwischen 2 Minuten und 5 Minuten.

6. Kühlvorrichtung nach einem der vorhergehenden Ansprüche, wobei die Steuereinheit programmiert ist, um den Betrieb der Energiequelle zu steuern, um die Talgdrüsen bei einer Geschwindigkeit zu erwärmen, die aus der Gruppe ausgewählt ist, die aus Folgendem besteht: zwischen 20 °C pro Minute und 30 °C pro Minute zwischen 30 °C pro Minute und 40 °C pro Minute, zwischen 40 °C pro Minute und 50 °C pro Minute und zwischen 50 °C pro Minute und 60 °C pro Minute; oder wobei die Steuereinheit programmiert ist, um den Betrieb der Energiequelle der Kühleinheit zu steuern, um die Talgdrüsen bei einer Geschwindigkeit zu erwärmen, die aus der Gruppe ausgewählt ist, die aus Folgendem besteht: zwischen 1 °C pro Sekunde und 2 °C pro Sekunde, zwischen 2 °C pro Sekunde und 3 °C pro Sekunde, zwischen 3 °C pro Sekunde und 4 °C pro Sekunde, zwischen 4 °C pro Sekunde und 5 °C pro Sekunde, zwischen 5 °C pro Sekunde und 6 °C pro Sekunde, zwischen 6 °C pro Sekunde und 7 °C pro Sekunde, zwischen 7 °C pro Sekunde und 8 °C pro Sekunde, zwischen 8 °C pro Sekunde und 9 °C pro Sekunde und zwischen 9 °C pro Sekunde und 10 °C pro Sekunde.

7. Kühlvorrichtung nach einem der vorhergehenden Ansprüche, wobei der Zeitraum, der erste Zeitraum oder der zweite Zeitraum aus der Gruppe ausgewählt ist, die aus Folgendem besteht: weniger als 5 Minuten, zwischen 5 Minuten und 10 Minuten, zwischen 10 Minuten und 15 Minuten, zwischen 15 Minuten und 20 Minuten, zwischen 20 Minuten und 25 Minuten, zwischen 25 Minuten und 30 Minuten, zwischen 30 Minuten und 35 Minuten, zwischen 35 Minuten und 40 Minuten, zwischen 40 Minuten und 45 Minuten, zwischen 45 Minuten und 50 Minuten, zwischen 50 Minuten und 55 Minuten und zwischen 55 Minuten und 60 Minuten.

8. Kühlvorrichtung nach einem der vorhergehenden Ansprüche, wobei die Steuereinheit programmiert ist, um den Betrieb der Kühleinheit zu steuern, um die eine oder mehreren Talgdrüsen unter eine Temperatur zu kühlen, die aus der Gruppe ausgewählt ist, die aus Folgendem besteht: -11 °C, -12 °C, -13 °C, -14 °C, -15 °C, -16 °C, -17 °C, -18 °C, -19 °C, -20 °C, -21 °C, -22 °C, -23 °C, -24 °C, -25 °C, -26 °C, -27 °C, -28 °C, -29 °C und -30 °C.

9. Kühlvorrichtung nach einem der vorhergehenden Ansprüche, wobei die Kühlvorrichtung ferner ein oder mehrere Abschnürungselemente umfasst und wobei die Steuereinheit ferner für Folgendes programmiert ist:
Festziehen des einen oder der mehreren Abschnürungselemente, um den Blutfluss in die lokale Region während der Kühlschritte zu reduzieren; und
Lösen des einen oder der mehreren Abschnürungselemente, um während des Erwärmungsschritts einen normalen Blutfluss in die lokale Region wiederherzustellen.

10. Kühlvorrichtung nach einem der vorhergehenden Ansprüche, wobei die Steuereinheit programmiert ist, um den Betrieb der Kühleinheit zu steuern, um die eine oder mehreren Talgdrüsen mehreren Kühl-Erwärmungs-Zyklen zu unterziehen; vorzugsweise, wobei die mehreren Kühl-Erwärmungs-Zyklen aus der Gruppe ausgewählt sind, die aus Folgendem besteht: 2, 3, 4, 5, 6, 7, 8, 9 und 10.

11. Kühlvorrichtung nach einem der vorhergehenden Ansprüche, ferner Folgendes umfassend:
eine Behandlungsschnittstelle in thermischer Kommunikation mit der Kühleinheit, wobei die Behandlungsschnittstelle für ein Aufbringen auf die lokalen Region angepasst und konfiguriert ist, wobei die Behandlungsschnittstelle eine Maske ist, die eine komplementäre Oberfläche aufweist, die im Wesentlichen einem menschlichen Gesicht entspricht, wobei die Behandlungsschnittstelle eine konvexe Oberfläche oder eine konkave Oberfläche aufweist; vorzugsweise ferner Folgendes umfassend:
ein Vakuum in Kommunikation mit der Behandlungsschnittstelle, wobei das Vakuum angepasst und konfiguriert ist, um einen reduzierten Druck zu generieren, um die Behandlungsschnittstelle gegen die lokale Region zu halten, und eine oder mehrere Rückmeldungsvorrichtungen in Kommunikation mit der Steuereinheit.

12. Kit, Folgendes umfassend:
die Kühlvorrichtung nach einem der vorhergehenden Ansprüche;
ein Zwischenmaterial; und
Anweisungen, um das Zwischenmaterial auf die lokale Region vor dem Aufbringen der Kühlvorrichtung aufzubringen.

13. Kit nach Anspruch 12, wobei das Zwischenmaterial eine Wärmeleitfähigkeit zwischen der Kühlvorrichtung verbessert, wobei das Zwischenmaterial ein Cryoprotektor ist; oder, wobei das Zwischenmaterial eines oder mehrere beinhaltet, die aus der Gruppe ausgewählt sind, die aus Folgendem besteht: Wasser, schwerem Wasser, Öl, Erdnussöl, Glycerin, Glycol, Polypropylenglycol (PPG), Polyethylenglycol (PEG), Propylenglycol, Ethylenglycol, Dimethylsulfoxid (DMSO), Alkohol, Ethanol, Propanol, Iso-Propanol, Carboxylpolyethylenpolymer, Hydroxyethylxylosepolymer, Carboxylmethylcellulose und Hydroxyethylcellulose (HEC).

14. Kühlvorrichtung wie zuvor definiert, die Energiequellen (522a, 522b) umfasst, die konfiguriert sind, um die Energie auf die Epidermis und/oder auf die Tiefe des Unterhautfettgewebes (145) zu fokussieren.

15. Kühlvorrichtung wie zuvor definiert, die Energiequellen (522a, 522b) umfasst, wobei die Kühleinheit (502) mehrere Öffnungen (526) beinhaltet, um zu ermöglichen, dass Energie von den Energiequellen aus der Kühlvorrichtung (500) austritt, oder wobei die Kühleinheit (502) aus einem Material hergestellt ist, das hinsichtlich der Wellenlänge der aufgebrachten Energie im Wesentlichen transparent ist.

## Revendications

1. Dispositif de refroidissement comprenant :
une unité de refroidissement ; et
une unité de commande programmée pour commander le fonctionnement de l'unité de refroidissement de façon à refroidir une ou plusieurs glandes sébacées à l'intérieur d'une région locale pendant une période et à une température en-dessous de 15 °C afin de perturber le rôle de la ou des glandes sébacées ; et
dans lequel l'unité de commande est programmée pour commander le fonctionnement de l'unité de refroidissement pour :
refroidir la ou les glandes sébacées pendant une première période ;
**caractérisé en ce que** l'unité de commande est en outre programmée pour
commander le fonctionnement de l'unité de refroidissement pour :
chauffer activement ou passivement la ou les glandes sébacées, de façon à maintenir le tissu adipeux sous-cutané dans la plage de température au-dessus de 25 °C, par l'un des éléments suivants :
a. l'actionnement d'une source d'énergie pour introduire de l'énergie dans la région locale ;
b. la commande de l'unité de refroidissement pour élever activement la température de l'unité de refroidissement ;
c. la suspension ou la réduction du fonctionnement de l'unité de refroidissement ;
d. une pluralité de cycles de refroidissement-chauffage ;
et refroidir la ou les glandes sébacées pendant une seconde période.

2. Dispositif de refroidissement, comprenant :
une unité de refroidissement ; et
une unité de commande programmée pour commander le fonctionnement de l'unité de refroidissement de façon à refroidir une ou plusieurs glandes sébacées à l'intérieur d'une région locale pendant une période et à une température en-dessous de 15 °C afin de perturber le rôle de la ou des glandes sébacées ;
**caractérisé en ce que** l'unité de commande est programmée pour commander le fonctionnement de l'unité de refroidissement afin d'employer des périodes de réchauffement passif, de réchauffement actif et/ou de commander les vitesses de refroidissement de manière à maintenir le tissu adipeux sous-cutané dans la plage de températures au-dessus de 25 °C.

3. Dispositif de refroidissement selon la revendication 1, dans lequel la source d'énergie est sélectionnée dans le groupe constitué d'une source de lumière cohérente, d'une source de lumière incohérente, d'une source de fluide chauffé, d'un dispositif de chauffage résistif, d'un générateur de micro-ondes qui produit de préférence des fréquences comprises entre environ 915 MHz et environ 2,45 GHz, et d'un générateur d'ultrasons qui produit de préférence des fréquences dans la plage de 300 kHz à 3 GHz.

4. Dispositif de refroidissement selon l'une quelconque des revendications précédentes, dans lequel l'unité de commande est programmée pour commander le fonctionnement de l'unité de refroidissement afin de refroidir la ou les glandes sébacées en dessous d'une température sélectionnée dans le groupe constitué de : 14 °C, 13 °C, 12 °C, 11 °C, 10 °C, 9 °C, 8 °C, 7 °C, 6 °C, 5 °C, 4 °C, 3 °C, 2 °C, 1 °C, 0 °C, -1 °C, -2 °C, -3 °C, -4 °C, -5 °C, - 6 °C, -7 °C, -8 °C, -9 °C et -10 °C ; et/ou, dans lequel l'unité de commande est programmée pour commander le fonctionnement de l'unité de refroidissement afin de refroidir les glandes sébacées à une vitesse sélectionnée dans le groupe constitué : d'entre 20 °C par minute et 30 °C par minute, d'entre 30 °C par minute et 40 °C par minute, d'entre 40 °C par minute et 50 °C par minute, et d'entre 50 °C par minute et 60 °C par minute ; et/ou, dans lequel l'unité de commande est programmée pour commander le fonctionnement de l'unité de refroidissement afin de refroidir les glandes sébacées à une vitesse sélectionnée dans le groupe constitué : d'entre 1 °C par seconde et 2 °C par seconde, d'entre 2 °C par seconde et 3 °C par seconde, d'entre 3 °C par seconde et 4 °C par seconde, d'entre 4 °C par seconde et 5 °C par seconde, d'entre 5 °C par seconde et 6 °C par seconde, d'entre 6 °C par seconde et 7 °C par seconde, d'entre 7 °C par seconde et 8 °C par seconde, d'entre 8 °C par seconde et 9 °C par seconde, et d'entre 9 °C par seconde et 10 °C par seconde.

5. Dispositif de refroidissement selon l'une quelconque des revendications précédentes, dans lequel la période, la première période ou la seconde période est sélectionnée dans le groupe constitué : de moins de 10 secondes, d'entre 10 secondes et 20 secondes, d'entre 20 secondes et 30 secondes, d'entre 30 secondes et 40 secondes, d'entre 40 secondes et 1 minute, d'entre 1 minute et 2 minutes et d'entre 2 minutes et 5 minutes.

6. Dispositif de refroidissement selon l'une quelconque des revendications précédentes, dans lequel l'unité de commande est programmée pour commander le fonctionnement de la source d'énergie afin de chauffer les glandes sébacées à une vitesse sélectionnée dans le groupe constitué : d'entre 20 °C par minute et 30 °C par minute, d'entre 30 °C par minute et 40 °C par minute, d'entre 40 °C par minute et 50 °C par minute, et d'entre 50 °C par minute et 60 °C par minute ; ou dans lequel l'unité de commande est programmée pour commander le fonctionnement de la source d'énergie de l'unité de refroidissement afin de chauffer les glandes sébacées à une vitesse sélectionnée dans le groupe constitué : d'entre 1 °C par seconde et 2 °C par seconde, d'entre 2 °C par seconde et 3 °C par seconde, d'entre 3 °C par seconde et 4 °C par seconde, d'entre 4 °C par seconde et 5 °C par seconde, d'entre 5 °C par seconde et 6 °C par seconde, d'entre 6 °C par seconde et 7 °C par seconde, d'entre 7 °C par seconde et 8 °C par seconde, d'entre 8 °C par seconde et 9 °C par seconde, et d'entre 9 °C par seconde et 10 °C par seconde.

7. Dispositif de refroidissement selon l'une quelconque des revendications précédentes, dans lequel la période, la première période ou la seconde période est sélectionnée dans le groupe constitué : de moins de 5 minutes, d'entre 5 minutes et 10 minutes, d'entre 10 minutes et 15 minutes, d'entre 15 minutes et 20 minutes, d'entre 20 minutes et 25 minutes, d'entre 25 minutes et 30 minutes, d'entre 30 minutes et 35 minutes, d'entre 35 minutes et 40 minutes, d'entre 40 minutes et 45 minutes, d'entre 45 minutes et 50 minutes, d'entre 50 minutes et 55 minutes et d'entre 55 minutes et 60 minutes.

8. Dispositif de refroidissement selon l'une quelconque des revendications précédentes, dans lequel l'unité de commande est programmée pour commander le fonctionnement de l'unité de refroidissement afin de refroidir la ou les glandes sébacées en dessous d'une température sélectionnée dans le groupe constitué de : -11 °C, -12 °C, -13 °C, -14 °C, - 15 °C, -16 °C, -17 °C, -18 °C, -19 °C, -20 °C, -21 °C, -22 °C, -23 °C, -24 °C, -25 °C, - 26 °C, -27 °C, -28 °C, -29 °C et -30 °C.

9. Dispositif de refroidissement selon l'une quelconque des revendications précédentes, dans lequel le dispositif de refroidissement comprend en outre un ou plusieurs éléments de constriction et dans lequel l'unité de commande est en outre programmée pour :
serrer le ou les éléments de constriction de façon à réduire le débit sanguin vers la région locale pendant les étapes de refroidissement ; et
desserrer le ou les éléments de constriction de façon à restaurer le débit sanguin normal vers la région locale pendant l'étape de chauffage.

10. Dispositif de refroidissement selon l'une quelconque des revendications précédentes, dans lequel l'unité de commande est programmée pour commander le fonctionnement de l'unité de refroidissement afin de soumettre la ou les glandes sébacées à une pluralité de cycles de refroidissement-chauffage ; de préférence, dans lequel la pluralité de cycles de refroidissement-chauffage sont sélectionnés dans le groupe constitué de : 2, 3, 4, 5, 6, 7, 8, 9 et 10.

11. Dispositif de refroidissement selon l'une quelconque des revendications précédentes, comprenant en outre :
une interface de traitement en communication thermique avec l'unité de refroidissement, l'interface de traitement étant adaptée et configurée pour une application sur la région locale, l'interface de traitement étant un masque ayant une surface complémentaire qui se rapproche sensiblement d'un visage humain, l'interface de traitement ayant une surface convexe ou une surface concave ; de préférence, comprenant en outre :
un vide en communication avec l'interface de traitement, le vide étant adapté et configuré pour générer une pression réduite de façon à maintenir l'interface de traitement contre la région locale, et un ou plusieurs dispositifs de rétroaction en communication avec l'unité de commande.

12. Kit comprenant :
le dispositif de refroidissement selon l'une quelconque des revendications précédentes ;
un matériau intermédiaire ; et
des instructions pour appliquer le matériau intermédiaire à la région locale avant l'application du dispositif de refroidissement.

13. Kit selon la revendication 12, dans lequel le matériau intermédiaire améliore la conductivité thermique entre le dispositif de refroidissement, dans lequel le matériau intermédiaire est un cryoprotecteur ; ou, dans lequel le matériau intermédiaire comporte un ou plusieurs matériaux sélectionnés dans le groupe constitué : d'eau, d'eau lourde, d'huile, d'huile d'arachide, de glycérol, de glycol, de polypropylène glycol (PPG), de polyéthylèneglycol (PEG), de propylène glycol, d'éthylène glycol, de diméthylsulfoxyde (DMSO), d'alcool, d'éthanol, de propanol, d'iso-propanol, de polymère carboxylpolyéthylène, de polymère hydroxyéthylxylose, de carboxylméthylcellulose et d'hydroxyéthylcellulose (HEC).

14. Dispositif de refroidissement tel que défini précédemment, comprenant des sources d'énergie (522a, 522b) configurées pour concentrer l'énergie sur l'épiderme et/ou au niveau de la profondeur du tissu adipeux sous-cutané (145).

15. Dispositif de refroidissement tel que défini précédemment, comprenant des sources d'énergie (522a, 522b), dans lequel l'unité de refroidissement (502) comporte une pluralité d'ouvertures (526) de façon à permettre à de l'énergie des sources d'énergie de sortir du dispositif de refroidissement (500), ou dans lequel l'unité de refroidissement (502) est faite d'un matériau qui est sensiblement transparent par rapport à la longueur d'onde de l'énergie appliquée.
